# EUROPEAN PATENT SPECIFICATION

(11) **EP 4 317 133 B1**
(45) Date of publication and mention of the grant of the patent: **15.04.2026**
(21) Application number: 22778848.6
(22) Date of filing: 28.03.2022
(51) Int. Cl.: C07D 207/34, A61P 1/16, A61P 5/40, A61P 7/04, A61P 9/04, A61P 9/10, A61P 9/12, A61P 13/12, A61K 31/40

(54) **CRYSTALLINE FORM OF PYRROLE AMIDE COMPOUND, PREPARATION METHOD THEREFOR AND USE THEREOF**
KRISTALLINE FORM EINER PYRROLAMIDVERBINDUNG, HERSTELLUNGSVERFAHREN DAFÜR UND VERWENDUNG DAVON
FORME CRISTALLINE D'UN COMPOSÉ DE PYRROLE AMIDE, SON PROCÉDÉ DE PRÉPARATION ET SON UTILISATION

(30) Priority: 30.03.2021 CN 202110339125
(43) Date of publication of application: 07.02.2024
(73) Proprietor: Sunshine Lake Pharma Co., Ltd., Dongguan, Guangdong 523000 (CN)
(72) Inventor: ZONG, Qiao, Dongguan, Guangdong 523871 (CN); CHEN, Liang, Dongguan, Guangdong 523871 (CN); ZUO, Yinglin, Dongguan, Guangdong 523871 (CN); WANG, Xiaojun, Dongguan, Guangdong 523871 (CN); WANG, Jiancheng, Dongguan, Guangdong 523871 (CN); ZHANG, Yingxun, Dongguan, Guangdong 523871 (CN)
(74) Representative: Hoffmann Eitle
(86) International application number: PCT/CN2022/083323
(87) International publication number: WO 2022/206666

(56) References cited:
- EP-A1- 3 190 102
- EP-A1- 3 950 674
- EP-A1- 4 332 091
- WO-A1-2021/078135
- CN-A- 101 006 052
- CN-A- 101 679 243
- CN-A- 102 186 817
- CN-A- 106 916 092
- CN-A- 112 707 854
- MIZUKI TAKAHASHI; OSAMU UBUKATA; TSUYOSHI HOMMA; YUSUKE ASOH; MASATOSHI HONZUMI; NORIYUKI HAYASHI; KEIJI SAITO; HIROYUKI TSURUOKA;: "Crystal structure of the mineralocorticoid receptor ligand‐binding domain in complex with a potent and selective nonsteroidal blocker, esaxerenone (CS‐3150)", FEBS LETTERS, ELSEVIER, AMSTERDAM., NL, vol. 594, no. 10, 19 February 2020 (2020-02-19), NL , pages 1615 - 1623, XP071256961, ISSN: 0014-5793, DOI: 10.1002/1873-3468.13746

## Description

### FIELD OF THE INVENTION

The invention belongs to the technical field of medicine, and relates to a crystalline form of a pyrrole amide compound and preparation methods and uses thereof, in particular relates to a crystalline form of (S)-1-(2-hydroxyethyl)-4-methyl-*N*-(3-fluoro-4-(methylsulfonyl)phenyl)-5-(2-(trifluoromethyl)phenyl)-1*H-*pyrrole-3-carboxamide (compound having formula (I)) and preparation methods and uses thereof. The present invention further relates to a pharmaceutical composition comprising the crystalline form.

### BACKGROUND ART

The mineralocorticoid receptor (MR) is an aldosterone-activated nuclear hormone receptor that regulates the expression of many genes involved in electrolyte homeostasis and cardiovascular disease. Increases in circulating aldosterone raise blood pressure through its effect on urinary sodium excretion, with potential effects on the brain, heart, and vascular system. In addition, hyperaldosteronism has been implicated in many disease processes leading to renal and cardiovascular disease. Although hyperaldosteronism is usually caused by aldosterone-producing adenomas, patients with resistant hypertension often have elevated aldosterone levels, which is often referred to as "aldosterone escape" and results from elevated serum potassium levels or residual AT1R activity. Hyperaldosteronism and aldosterone escape typically result in increased MR activity, and MR antagonists have been shown to be effective antihypertensive agents and are also effective in the treatment of heart failure and primary hyperaldosteronism. In addition, MR antagonists have also been shown to be effective in preclinical models of renal disease and may be used in combination with standard therapy to reduce proteinuria in patients with renal disease, such as chronic kidney disease, including diabetic nephropathy.

Drug polymorphism is a common phenomenon in drug development and an important factor affecting the quality of drugs. Different crystalline forms of the same drug may have significant differences in appearance, solubility, melting point, dissolution, bioavailability, *etc.,* and may have different effects on the stability, bioavailability and efficacy of the drug. Therefore, the drug should be fully considered the problem of polymorphism in drug research and development.

### SUMMARY

WO2021078135A1 discloses a class of pyrrole amide compounds that can be used as mineralocorticoid receptor antagonists, and specifically discloses the compound (*S*)-1-(2-hydroxyethyl)-4-methyl-*N*-(3-fluoro-4-(methylsulfonyl)phenyl)-5-(2-(trifluoromethyl)phenyl )-1H-pyrrole-3-carboxamide (see Example 2 of this patent application), preparation methods and uses thereof. The inventors have found through research that the pyrrole amide compound has mineralocorticoid receptor (MR) antagonistic effect, and can be used to treat, prevent or alleviate diseases or conditions such as hyperaldosteronism, hypertension, chronic heart failure, sequelae of myocardial infarction, liver cirrhosis, nonalcoholic steatohepatitis, chronic kidney disease, diabetic nephropathy, renal failure, fibrosis and/or stroke in patients. At the same time, the inventors conducted further research on the compound of Example 2 of the patent application (*i.e.,* the compound having formula (I) of the present application), and found that the compound is a white solid, usually in an amorphous form, and tried various methods, but it was difficult to obtain a pure crystalline form with a single microstructure. Through unremitting efforts and countless attempts, the inventors finally obtained a single crystalline form of the compound having formula (I), that is, the crystalline form I described in the present invention; and unexpectedly found that the crystalline form I described in the present invention has good pharmacokinetic properties, high stability, low hygroscopicity, and good solubility, and is suitable for pharmaceutical use.

The present invention provides a crystalline form of the compound having formula (I) and use thereof, wherein the crystalline form has good stability, pharmacokinetics, low hygroscopicity and other properties, thereby having good druggability.

Specifically, the present invention relates to a crystalline form I of the compound having formula (I) or a pharmaceutical composition comprising the crystalline form I, and uses of the crystalline form I or the pharmaceutical composition as a mineralocorticoid antagonist, and/or in the manufacture of a medicament for treating or preventing diseases related to mineralocorticoids. The crystalline form of the present invention may also be in the form of solvates, such as hydrates.

In one aspect, the present invention provides a crystalline form of the compound having formula (I),

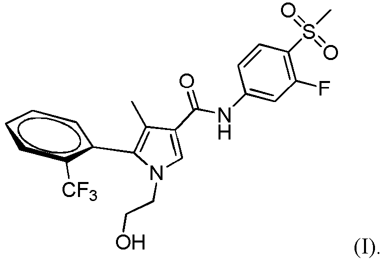

The crystal form of the compound having formula (I) described in the present invention is crystalline form I.

The crystalline form I of the present invention is characterized in that the X-ray powder diffraction pattern of the crystalline form I has diffraction peaks at the following 2θ angles: 14.45°± 0.2°, 17.04°± 0.2°, 19.35°± 0.2°, 22.51°± 0.2°, 24.78°± 0.2°, wherein the X-ray powder diffraction pattern was obtained using Cu-Kα radiation.

In some embodiments, the crystalline form I of the present invention is characterized in that the X-ray powder diffraction pattern of the crystalline form I also has diffraction peaks at at least one of the following 2θ angles: 6.89°± 0.2°, 16.42°± 0.2°, 18.31°± 0.2°, 22.25°± 0.2°, 23.06°± 0.2°.

In some embodiments, the crystalline form I of the present invention is characterized in that the X-ray powder diffraction pattern of the crystalline form I has diffraction peaks at the following 2θ angles: 6.89°±0.2°, 14.45°±0.2°, 16.42°± 0.2°, 17.04°± 0.2°, 18.31°± 0.2°, 19.35°± 0.2°, 22.25°± 0.2°, 22.51°± 0.2°, 23.06°± 0.2°, 24.78°± 0.2°.

In some embodiments, the crystalline form I of the present invention is characterized in that the X-ray powder diffraction pattern of the crystalline form I also has diffraction peaks at at least one of the following 2θ angles: 12.25°±0.2°, 13.70°±0.2°, 13.99°± 0.2°, 20.65°± 0.2°, 21.83°± 0.2°, 21.95°± 0.2°, 24.59°± 0.2°, 24.93°± 0.2°, 25.77°± 0.2°, 26.98°± 0.2°.

In some embodiments, the crystalline form I of the present invention is characterized in that the X-ray powder diffraction pattern of the crystalline form I has diffraction peaks at the following 2θ angles: 6.89°± 0.2°, 12.25°± 0.2°, 13.70°± 0.2°, 13.99°± 0.2°, 14.45°± 0.2°, 16.42°± 0.2°, 17.04°± 0.2°, 18.31°± 0.2°, 19.35°± 0.2°, 20.65°± 0.2°, 21.83°± 0.2°, 21.95°± 0.2°, 22.25°± 0.2°, 22.51°± 0.2°, 23.06°± 0.2°, 24.59°± 0.2°, 24.78°± 0.2°, 24.93°± 0.2°, 25.77°± 0.2°, 26.98°± 0.2°.

In some embodiments, the crystalline form I of the present invention is characterized in that the X-ray powder diffraction pattern of the crystalline form I also has diffraction peaks at at least one of the following 2θ angles: 13.58°±0.2°, 15.19°±0.2°, 18.00°± 0.2°, 19.97°± 0.2°, 23.16°± 0.2°, 29.11°± 0.2°, 29.33°± 0.2°, 31.96°± 0.2°, 32.42°± 0.2°, 33.74°± 0.2°.

In some embodiments, the crystalline form I of the present invention is characterized in that the X-ray powder diffraction pattern of the crystalline form I has diffraction peaks at the following 2θ angles: 6.89°± 0.2°, 12.25°± 0.2°, 13.58°± 0.2°, 13.70°± 0.2°, 13.99°± 0.2°, 14.45°± 0.2°, 15.19°± 0.2°, 16.42°± 0.2°, 17.04°± 0.2°, 18.00°± 0.2°, 18.31°± 0.2°, 19.35°± 0.2°, 19.97°± 0.2°, 20.65°± 0.2°, 21.83°± 0.2°, 21.95°± 0.2°, 22.25°± 0.2°, 22.51°± 0.2°, 23.06°± 0.2°, 23.16°± 0.2°, 24.59°± 0.2°, 24.78°± 0.2°, 24.93°± 0.2°, 25.77°± 0.2°, 26.98°± 0.2°, 29.11°± 0.2°, 29.33°± 0.2°, 31.96°± 0.2°, 32.42°± 0.2°, 33.74°± 0.2°.

In some embodiments, the crystalline form I of the present invention is characterized in that the X-ray powder diffraction pattern of the crystalline form I also has diffraction peaks at at least one of the following 2θ angles: 10.71°± 0.2°, 11.31°± 0.2°, 20.32°± 0.2°, 21.48°± 0.2°, 23.31°± 0.2°, 27.46°± 0.2°, 27.75°± 0.2°, 28.15°± 0.2°, 29.82°± 0.2°, 30.51°± 0.2°, 30.83°± 0.2°, 32.87°± 0.2°, 34.78°± 0.2°, 35.87°± 0.2°, 36.25°± 0.2°, 36.52°± 0.2°, 37.33°± 0.2°, 37.98°± 0.2°, 38.43°± 0.2°, 39.08°± 0.2°, 39.34°± 0.2°, 40.16°± 0.2°, 40.78°± 0.2°, 41.94°± 0.2°, 42.18°± 0.2°, 43.61°± 0.2°, 44.35°± 0.2°, 44.66°± 0.2°, 45.39°± 0.2°, 47.66°± 0.2°.

In some embodiments, the crystalline form I of the present invention is characterized in that the X-ray powder diffraction pattern of the crystalline form I has diffraction peaks at the following 2θ angles: 6.89°± 0.2°, 10.71°± 0.2°, 12.25°± 0.2°, 13.58°± 0.2°, 13.70°± 0.2°, 13.99°± 0.2°, 14.45°± 0.2°, 15.19°± 0.2°, 16.42°± 0.2°, 17.04°± 0.2°, 18.00°± 0.2°, 18.31°± 0.2°, 19.35°± 0.2°, 19.97°± 0.2°, 20.32°± 0.2°, 20.65°± 0.2°, 21.83°± 0.2°, 21.95°± 0.2°, 22.25°± 0.2°, 22.51°± 0.2°, 23.06°± 0.2°, 23.16°± 0.2°, 23.31°± 0.2°, 24.59°± 0.2°, 24.78°± 0.2°, 24.93°± 0.2°, 25.77°± 0.2°, 26.98°± 0.2°, 27.46°± 0.2°, 27.75°± 0.2°, 29.11°± 0.2°, 29.33°± 0.2°, 30.51°± 0.2°, 30.83°± 0.2°, 31.96°± 0.2°, 32.42°± 0.2°, 33.74°± 0.2°, 35.87°± 0.2°, 36.25°± 0.2°, 38.43°± 0.2°, 40.16°± 0.2°, 41.94°± 0.2°, 42.18°± 0.2°, 43.61°± 0.2°.

In some embodiments, the crystalline form I of the present invention is characterized in that the X-ray powder diffraction pattern of the crystalline form I has diffraction peaks at the following 2θ angles: 6.89°± 0.2°, 10.71°± 0.2°, 11.31°± 0.2°, 12.25°± 0.2°, 13.58°± 0.2°, 13.70°± 0.2°, 13.99°± 0.2°, 14.45°± 0.2°, 15.19°± 0.2°, 16.42°± 0.2°, 17.04°± 0.2°, 18.00°± 0.2°, 18.31°± 0.2°, 19.35°± 0.2°, 19.97°± 0.2°, 20.32°± 0.2°, 20.65°± 0.2°, 21.48°± 0.2°, 21.83°± 0.2°, 21.95°± 0.2°, 22.25°± 0.2°, 22.51°± 0.2°, 23.06°± 0.2°, 23.16°± 0.2°, 23.31°± 0.2°, 24.59°± 0.2°, 24.78°± 0.2°, 24.93°± 0.2°, 25.77°± 0.2°, 26.98°± 0.2°, 27.46°± 0.2°, 27.75°± 0.2°, 28.15°± 0.2°, 29.11°± 0.2°, 29.33°± 0.2°, 29.82°± 0.2°, 30.51°± 0.2°, 30.83°± 0.2°, 31.96°± 0.2°, 32.42°± 0.2°, 32.87°± 0.2°, 33.74°± 0.2°, 34.77°± 0.2°, 35.87°± 0.2°, 36.25°± 0.2°, 36.52°± 0.2°, 37.33°± 0.2°, 37.98°± 0.2°, 38.43°± 0.2°, 39.08°± 0.2°, 39.34°± 0.2°, 40.16°± 0.2°, 40.78°± 0.2°, 41.94°± 0.2°, 42.18°± 0.2°, 43.61°± 0.2°, 44.35°± 0.2°, 44.66°± 0.2°, 45.39°± 0.2°, 47.66°± 0.2°.

In some embodiments, the crystalline form I of the present invention is characterized in that the X-ray powder diffraction pattern of the crystalline form I has diffraction peaks at the following 2θ angles: 6.89°± 0.2°, 10.71°± 0.2°, 11.31°± 0.2°, 12.25°± 0.2°, 13.58°± 0.2°, 13.70°± 0.2°, 13.99°± 0.2°, 14.45°± 0.2°, 15.19°± 0.2°, 16.42°± 0.2°, 17.04°± 0.2°, 18.00°± 0.2°, 18.31°± 0.2°, 19.35°± 0.2°, 19.97°± 0.2°, 20.32°± 0.2°, 20.65°± 0.2°, 21.48°± 0.2°, 21.83°± 0.2°, 21.95°± 0.2°, 22.25°± 0.2°, 22.51°± 0.2°, 23.06°± 0.2°, 23.16°± 0.2°, 23.31°± 0.2°, 24.59°± 0.2°, 24.78°± 0.2°, 24.93°± 0.2°, 25.77°± 0.2°, 26.22°± 0.2°, 26.98°± 0.2°, 27.46°± 0.2°, 27.75°± 0.2°, 28.15°± 0.2°, 29.11°± 0.2°, 29.33°± 0.2°, 29.82°± 0.2°, 30.51°± 0.2°, 30.83°± 0.2°, 31.44°± 0.2°, 31.96°± 0.2°, 32.42°± 0.2°, 32.87°± 0.2°, 33.74°± 0.2°, 34.77°± 0.2°, 35.87°± 0.2°, 36.25°± 0.2°, 36.52°± 0.2°, 37.33°± 0.2°, 37.98°± 0.2°, 38.43°± 0.2°, 39.08°± 0.2°, 39.34°± 0.2°, 40.16°± 0.2°, 40.78°± 0.2°, 41.47°± 0.2°, 41.94°± 0.2°, 42.18°± 0.2°, 42.71°± 0.2°, 43.61°± 0.2°, 44.35°± 0.2°, 44.66°± 0.2°, 45.05°± 0.2°, 45.39°± 0.2°, 45.88°± 0.2°, 46.69°± 0.2°, 47.21°± 0.2°, 47.66°± 0.2°, 48.32°± 0.2°, 48.97°± 0.2°, 49.38°± 0.2°, 50.35°± 0.2°, 50.65°± 0.2°, 51.97°± 0.2°, 53.24°± 0.2°, 55.00°± 0.2°, 56.23°± 0.2°, 58.12°± 0.2°.

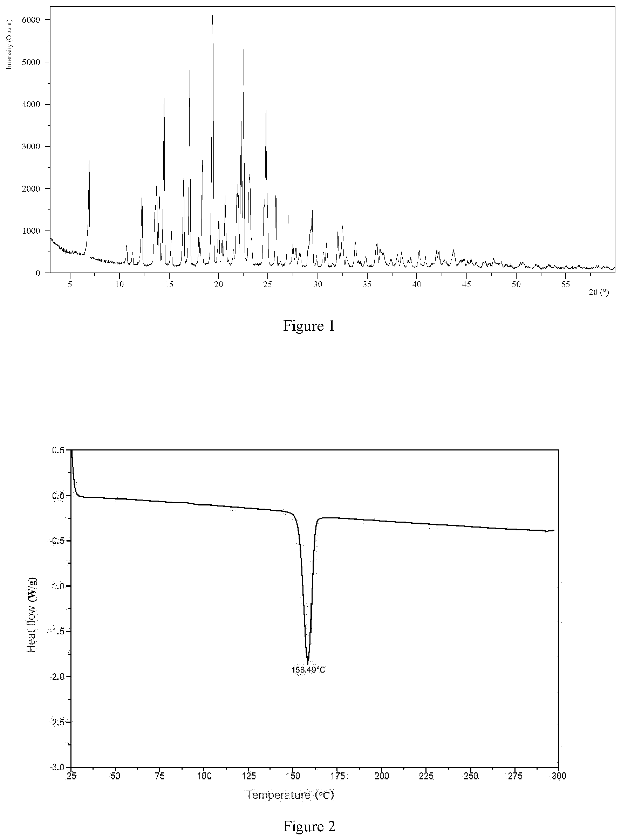

In some embodiments, the crystalline form I of the present invention is characterized in that the crystalline form I has an X-ray powder diffraction pattern substantially as shown in Figure 1.

In some embodiments, the crystalline form I of the present invention is characterized in that the differential scanning calorimetry diagram of the crystalline form I comprises an endothermic peak at 158.49°C ± 3°C, wherein the differential scanning calorimetry diagram was obtained from room temperature to about 300°C using a linear heating device at 10°C/min.

In some embodiments, the crystalline form I of the present invention is characterized in that the crystalline form I has a differential scanning calorimetry diagram substantially as shown in Figure 2.

In some embodiments, the crystalline form I of the present invention is characterized in that, when the crystalline form I is heated to about 200.13°C, the weight loss is about 0.034%, and there is an error tolerance of ± 0.1%.

In another aspect, the present invention relates to a pharmaceutical composition, which comprises the crystalline form I of the present invention, and a pharmaceutically acceptable carrier, excipient, diluent, adjuvant or a combination thereof.

In some embodiments, the pharmaceutical composition of the present invention further comprises one or more other active ingredients selected from sodium-glucose cotransporter 2 (SGLT-2) inhibitors, soluble guanylate cyclase (sGC) activators, soluble guanylate cyclase (sGC) stimulators, angiotensin converting enzyme (ACE) inhibitors, renin inhibitors, angiotensin II receptor antagonists, β-blockers, acetylsalicylic acid, diuretics, calcium antagonists agents, statins, digitalis derivatives, calcium sensitizers, nitrates and antithrombotics.

In one aspect, the present invention relates to use of the crystalline form I or the pharmaceutical composition in the manufacture of a medicament for treating, preventing or alleviating the following disease or condition in patients: diabetic nephropathy, hyperaldosteronism, hypertension, heart failure, sequelae of myocardial infarction, liver cirrhosis, nonalcoholic steatohepatitis, chronic kidney disease, fibrosis, renal failure, or stroke.

In some embodiments, the heart failure is chronic heart failure.

In one aspect, the present invention relates to the crystalline form I or the pharmaceutical composition for use in treating, preventing or alleviating the following disease or condition in patients: diabetic nephropathy, hyperaldosteronism, hypertension, heart failure, sequelae of myocardial infarction, liver cirrhosis, nonalcoholic steatohepatitis, chronic kidney disease, fibrosis, renal failure, or stroke.

In another aspect, the present invention relates to use of the crystalline form I or the pharmaceutical composition in the manufacture of a medicament as a mineralocorticoid receptor antagonist.

In another aspect, the present invention relates to the crystalline form I or the pharmaceutical composition for use as a mineralocorticoid receptor antagonist.

In one aspect, the present invention provides a method for preparing the crystalline form I of the compound having Formula (I) as described in the present invention, comprising: 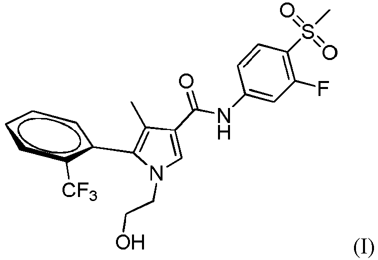
step 1): the compound having Formula (I) is added into solvent 1, and the resulting suspension is stirred until dissolved;
step 2): solvent 2 is added dropwise to the clear solution in step 1); and
step 3): the resulting mixture is stirred and crystallized.

Specifically, the crystalline form I has the meaning described in the present invention.

In some embodiments, in the method of the present invention, the solvent 1 is an ester solvent or an alcohol solvent. In other embodiments, the ester solvent of the present invention is ethyl acetate, n-propyl acetate, isopropyl acetate, *tert*-butyl acetate or methyl acetate, and the alcohol solvent is methanol, ethanol or isopropanol. Preferably, the ester solvent of the present invention is isopropyl acetate, and the alcohol solvent is ethanol.

In some embodiments, in the method of the present invention, the solvent 2 is n-hexane, cyclohexane, n-heptane, xylene, toluene or water; preferably, the solvent 2 is n-heptane, toluene or water.

In some embodiments, in the method of the present invention, the solvent 1 is isopropyl acetate, and the solvent 2 is n-heptane or toluene; or, the solvent 1 is ethanol, and the solvent 2 is water.

In some embodiments, in the method of the present invention, based on the mass of the compound having Formula (I), the volume of the solvent 1 is 3-5 mL/g, that is, for every gram of compound having Formula (I), the amount of solvent 1 is 3 mL-5 mL. Preferably, in the method of the present invention, based on the mass of the compound having Formula (I), the volume of the solvent 1 is 3 mL/g, 4 mL/g or 5 mL/g.

In some embodiments, in the method of the present invention, based on the mass of the compound having Formula (I), the volume of the solvent 2 is 3-15 mL/g, that is, for every gram of compound having Formula (I), the amount of solvent 2 is 3 mL-15 mL. Preferably, in the method of the present invention, based on the mass of the compound having Formula (I), the volume of the solvent 2 is 3-4 mL/g, 4.5-7 mL/g or 12-15 mL/g.

In some embodiments, the solvent 2 described in the present invention can be added at one time, or can be added in multiple batches. In some embodiments, the solvent 2 of the present invention can be added in two batches; in other embodiments, the solvent 2 of the present invention is added in two batches, most of the solvent 2 is added for the first time, and the remaining portion of the solvent 2 is added after stirring for a period of time.

In some embodiments, the solvent 2 can be added dropwise; in other embodiments, the solvent 2 can be added slowly dropwise.

In some embodiments, in the method of the present invention, in the step 1), the suspension is stirred at room temperature or heated to a certain temperature until clear, wherein the certain temperature is 40°C-80°C, preferably 50°C-70°C, more preferably 50°C-60°C. In other embodiments, in the step 1), the suspension is stirred at room temperature, 55 °C or 60 °C until clear. Wherein, the error tolerance of the temperature is ± 5 °C.

In some embodiments, in the method of the present invention, in the step 3), the stirring and crystallization is carried out at room temperature or at a certain temperature, wherein the certain temperature is 40°C-80°C, preferably 50°C-70°C, more preferably 50°C-60°C. In other embodiments, in the step 3), the stirring and crystallization is carried out at room temperature, 55 °C or 60 °C. Wherein, the error tolerance of the temperature is ± 5 °C.

In some embodiments, the raw material in step 1) of the present invention-the compound having Formula (I) can be its amorphous form, any suitable crystalline form, or any combination thereof.

The solvent used in the production method of the crystalline form described in the present invention is not particularly restricted, and any solvent which dissolves the starting material to a degree and does not affect its properties is contained in the present invention. Additionally, many similar modifications in the art, equivalent replacements, or solvent, solvent composition and the solvent composition with different proportions which are equivalent to those described in the invention, all are deemed to be included in the present invention. The present invention gives the preferred solvent for each reaction step.

The preparation of the crystalline form of the present invention will be described in detail in the examples section.

The preparation method of the crystalline form I provided by the invention is simple to operate, has good reproducibility, and is easy to control the process. It has a stable process method, high yield, high purity of the prepared crystal form I, and is suitable for industrial production.

Meanwhile, the present invention provides pharmacokinetic experiments of the crystalline form, solubility experiments, stability experiments, hygroscopicity experiments and the like.

Stability experiments have proved that the crystalline form of the present invention, especially the crystalline form I, has good stability, and can well avoid changes in bioavailability and drug efficacy during storage and development of the drug.

Experiments have proved that the crystalline form of the present invention, especially the crystalline form I, has better solubility, which is beneficial to improving drug efficacy and reducing drug loading. At the same time, the crystalline form of the present invention, especially the crystalline form I, has better biological activity.

In addition, according to the results of the hygroscopicity experiment, the crystalline form I of the invention is not susceptible to deliquescence due to the influence of high humidity, which is convenient for the long-term storage of the drug.

In summary, the crystalline form I of the invention has good biological activity, good solubility and high stability, and is suitable for pharmaceutical use. The method of the invention is stable, simple and easy to control and has high yield, and is suitable for industrialized production.

### DESCRIPTION OF THE DRAWINGS

Figure 1 is an X-ray powder diffraction (XRPD) pattern of the crystalline form I of the compound having Formula (I).
Figure 2 is a differential scanning calorimetry (DSC) diagram of the crystalline form I of the compound having Formula (I).
Figure 3 is a thermal gravimetric analysis (TGA) diagram of the crystalline form I of the compound having Formula (I).
Figure 4 is a dynamic vapour adsorption (DVS) diagram of the crystalline form I of the compound having Formula (I).
Figure 5 is an X-ray powder diffraction (XRPD) pattern of the amorphous form of the compound having Formula (I).
Figure 6 is a dynamic vapour adsorption (DVS) diagram of the amorphous form of the compound having Formula (I).

### EXAMPLES

### DEFINITIONS AND GENERAL TERMINOLOGY

Unless otherwise indicated, all technical and scientific terms used in the present invention have the same meaning as commonly understood by one of ordinary skill in the art to which this invention pertains. Although any methods and materials similar or identical to those described herein may be used in the practice or testing of the invention, but the methods, apparatus and materials described in the invention are preferred.

"Crystal form" or "crystalline form" refers to a solid having a highly regular chemical structure, including, but not limited to, mono- or multi-component crystals, and/or polymorphic compounds of compounds, solvates, hydrates, clathrates, eutectic, salts, solvates of the salts, hydrates of the salts. The crystalline form of the material can be obtained by a number of methods known in the field. Such methods include, but are not limited to, melt crystallization, melt cooling, solvent crystallization, crystallization in defined space, for example, in nanopores or capillaries, on a surface or template, for example, on a polymer, crystallization in the presence of additives such as co-crystallized anti-molecules, desolvation, dehydration, rapid evaporation, rapid cooling, slow cooling, vapor diffusion, sublimation, reaction crystallization, anti-solvent addition, grinding and solvent drop milling, *etc.*

"Solvent" refers to a substance (typically a liquid) that is capable of completely or partially dissolving another substance (typically a solid). Solvents for use in the practice of this invention include, but are not limited to, water, acetic acid, acetone, acetonitrile, benzene, chloroform, carbon tetrachloride, dichloromethane, dimethylsulfoxide, 1,4-dioxane, ethanol, ethyl acetate, butanol, t-butanol, N, N-dimethylacetamide, *N,* N-dimethylformamide, formamide, formic acid, heptane, hexane, isopropanol, methanol, methyl ethyl ketone, mesitylene, nitromethane, polyethylene glycol, propanol, pyridine, tetrahydrofuran, toluene, xylene, mixtures thereof, and the like.

"Solvate" refers to a compound having a solvent on a surface, in a lattice or having a solvent on a surface and in a lattice. The solvent can be water, acetic acid, acetone, acetonitrile, benzene, chloroform, carbon tetrachloride, dichloromethane, dimethylsulfoxide, 1,4-dioxane, ethanol, ethyl acetate, butanol, t-butanol, *N,* N-dimethylacetamide, *N,* N-dimethylformamide, formamide, formic acid, heptane, hexane, isopropanol, methanol, methyl ethyl ketone, methyl pyrrolidone, mesitylene, nitromethane, polyethylene glycol, propanol, pyridine, tetrahydrofuran, toluene, xylene, mixtures thereof, and the like. A specific example of the solvate is a hydrate in which the solvent on the surface, in the lattice or the solvent on the surface and in the lattice is water. Hydrates may or may not have other solvents besides water, on the surface of the substance, in the lattice, or both.

Crystalline form can be identified by a variety of technical means, such as X-ray powder diffraction (XRPD), infrared absorption spectroscopy (IR), melting point method, differential scanning calorimetry (DSC), thermogravimetric analysis (TGA), nuclear magnetic resonance, Raman spectroscopy, X-ray single crystal diffraction, dissolution calorimetry, scanning electron microscopy (SEM), quantitative analysis, solubility and dissolution rate.

X-ray powder diffraction (XRPD) can detect changes in crystalline form, crystallinity, crystalline state and other information, and is a common means for identifying crystalline form. The peak position of the XRPD pattern primarily depends on the structure of the crystalline form and is relatively insensitive to the experimental details, and its relative peak height depends on many factors associated with sample preparation and instrument geometry. Thus, in some embodiments, the crystalline form of the present invention is characterized by an XRPD pattern having certain peak positions, which is substantially as shown in the XRPD pattern provided in the drawings of the present invention. At the same time, the 2θ of the XRPD pattern can be measured with an experimental error. The measurement of 2θ of the XRPD pattern may be slightly different between the different instruments and the different samples. Therefore, the value of 2θ cannot be regarded as absolute. According to the condition of the instrument used in this test, the diffraction peak has an error tolerance of ± 0.2°.

Differential Scanning Calorimetry (DSC) is a technique of measuring the energy difference between a sample and an inert reference (commonly used α-Al₂O₃) with temperature by continuously heating or cooling under program control. The endothermic peak of the DSC diagram depends on many factors associated with sample preparation and instrument geometry, while the peak position is relatively insensitive to experimental details. Thus, in some embodiments, the crystalline form of the present invention is characterized by an DCS diagram having certain peak positions, which is substantially as shown in the DCS diagram provided in the drawings of the present invention. At the same time, the DCS diagram can be measured with an experimental error. The peak position and peak value of DCS diagram may be slightly different between the different instruments and the different samples. Therefore, the peak position or the peak value of the DSC endothermic peak cannot be regarded as absolute. According to the condition of the instrument used in this test, the endothermic peak has an error tolerance of ± 3°C.

Thermogravimetric analysis (TGA) is a technique for measuring the quality of a substance with temperature under the control of a program. It is suitable for examining the loss of solvent in the crystal or the process of sublimation and decomposition of the sample. It can be presumed that the crystal contains crystalline water or crystallization solvent. The mass change of the TGA diagram shown depends on a number of factors such as the sample preparation and the instrument. The mass change detected by TGA varies slightly between different instruments and different samples. According to the condition of the instrument used in the experiments of the present invention, there is a ± 0.1% error tolerance for the mass change.

In the context of the present invention, the 2θ values in the X-ray powder diffraction pattern are in degrees (°).

The term "substantially as shown in the figure" refers to at least 50%, or at least 60%, or at least 70%, or at least 80%, or at least 90%, or at least 95%, or at least 99% of the peaks are shown in the X-ray powder diffraction pattern or DSC diagram or Raman spectra pattern or infrared spectra pattern.

The "peak" refers to a feature that a person skilled in the art can recognize without belonging to background noise when referring to a spectrum or/and data that appears in the figure.

The present invention relates to crystalline forms of compound having Formula (I), for example, crystalline form I, which exists in a substantially pure crystalline form.

"Substantially pure" means that a crystalline form is substantially free of another or more crystalline forms, that is, the purity of the crystalline form is at least 80%, or at least 85%, or at least 90%, or at least 93%, or at least 95%, or at least 98%, or at least 99%, or at least 99.5%, or at least 99.6%, or at least 99.7%, or at least 99.8%, or at least 99.9%, or crystalline form containing other crystalline form. The percentage of the other crystalline forms in the total volume or total weight of the crystalline form is less than 20%, or less than 10%, or less than 5%, or less than 3%, or less than 1%, or less than 0.5%, or less than 0.1%, or less than 0.01%.

"Substantially free" means that the percentage of one or more other crystalline forms in the total volume or total weight of the crystalline form is less than 20%, or less than 10%, or less than 5%, or less than 4%, or less than 3%, or less than 2%, or less than 1%, or less than 0.5%, or less than 0.1%, or less than 0.01%.

The "relative intensity" (or "relative peak height") in the XRPD diagram means the ratio of the intensity of the other peaks to the intensity of the first strong peak when the intensity of the first strong peak in all the diffraction peaks of the X-ray powder diffraction pattern (XRPD) is 100%.

In the context of the present invention, when used or whether or not used the word, such as "about" or "approximately", it means that within a given value or range of 10% or less, appropriately within 5%, especially within 1%. Or, for those of ordinary skill in the art, the term "about" or "approximately" means within an acceptable standard error range of the mean value. When a number with an N value is made public, any number within N +/- 1%, N +/- 2%, N +/- 3%, N +/- 5%, N +/- 7%, N +/- 8%, or N +/- 10% will be opened clearly, wherein "+/-" means plus or minus.

In the present invention, "room temperature" refers to a temperature from about 10 °C to about 40 °C. In some embodiments, "room temperature" refers to a temperature from about 20 °C to about 30 °C; in other embodiments, "room temperature" refers to 20 °C, 22.5 °C, 25 °C, 27.5 °C, *etc.*

### PHARMACEUTICAL COMPOSITIONS, FORMULATIONS, ADMINISTRATION AND USE OF THE CRYSTALLINE FORMS OF THE PRESENT INVENTION

The characteristics of the pharmaceutical composition of the present invention include the crystalline form of the compound having Formula (I) and pharmaceutically acceptable carriers, adjuvants, or excipients. The amount of the crystalline form of the compound in the pharmaceutical composition of the present invention can effectively and detectably treat or alleviate mineralocorticoid-related diseases in patients.

As described above, the pharmaceutically acceptable composition of the present invention further comprises a pharmaceutically acceptable carrier, an adjuvant, or a vehicle, which, as used herein, includes any and all solvents, diluents, or other liquid vehicle, dispersion or suspension aids, surface active agents, isotonic agents, thickening or emulsifying agents, preservatives, solid binders, lubricants and the like, as suited to the particular dosage form desired. As described in the following: In Remington: The Science and Practice of Pharmacy, 21st edition, 2005, ed. D.B. Troy, Lippincott Williams& Wilkins, Philadelphia, and Encyclopedia of Pharmaceutical Technology, eds. J. Swarbrick and J. C. Boylan, 1988-1999, Marcel Dekker, New York, discloses various carriers used in formulating pharmaceutically acceptable compositions and known techniques for the preparation thereof. Except insofar as any conventional carrier medium incompatible with the crystalline form of the compound disclosed herein, such as by producing any undesirable biological effect or otherwise interacting in a deleterious manner with any other components of the pharmaceutically acceptable composition, its use is contemplated to be within the scope of this invention.

Some non-limiting examples of materials which can serve as pharmaceutically acceptable carriers include ion exchangers; aluminium; aluminum stearate; lecithin; serum proteins such as human serum albumin; buffer substances such as phosphates; glycine; sorbic acid; potassium sorbate; partial glyceride mixtures of saturated vegetable fatty acids; water; salts or electrolytes such as protamine sulfate, disodium hydrogen phosphate, potassium hydrogen phosphate, sodium chloride and zinc salts; colloidal silica; magnesium trisilicate; polyvinyl pyrrolidone; polyacrylates; waxes; polyethylene-polyoxypropylene-block polymers; wool fat; sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil, safflower oil, sesame oil, olive oil, corn oil and soybean oil; glycols such as propylene glycol and polyethylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol; phosphate buffer solutions; as well as other non-toxic compatible lubricants such as sodium lauryl sulfate and magnesium stearate, as well as coloring agents, releasing agents, coating agents, sweetening, flavoring and perfuming agents, preservatives and antioxidants.

The pharmaceutical compositions disclosed herein can be capsules, tablets, pills, powders, granules and aqueous suspensions or solutions; it can be administered orally, parenterally, by inhalation spray, topically, rectally, nasally, buccally, vaginally or via an implanted reservoir.

Oral administration can be administered in the following forms: tablets, pills, capsules, dispersible powders, granules or suspensions, syrups, elixirs, *etc*.; external administration can be administered in the following forms: ointment, gel, medicated adhesive tape, *etc.*

The crystalline forms of the present invention are preferably formulated in dosage unit form according to the formulation to reduce dosage and uniformity of dosage. The term "dosage unit form" herein refers to a physically dispersed unit in which a patient obtains the appropriate treatment. However, it should be understood that the daily general use of the compound having Formula (I) of the present invention or its crystalline form or the pharmaceutical composition of the present invention will be determined by the attending physician on the basis of a reliable medical range judgment. The specific effective dosage level for any particular patient or organism will depend on a number of factors including the severity of the disease and condition being treated, the activity of the particular compound or crystalline form thereof, the particular composition used, the age, weight, health, sex and eating habits of the patient, time of administration, route of administration and excretion rate of the particular compound or its crystalline form used, duration of treatment, drug use in combination or in combination with a specific compound or its crystalline form, and other well known factors in the field of pharmacy.

The effective dosage of the active ingredient used may vary with the compound used or its crystalline form, the mode of administration and the severity of the disease to be treated. However, generally satisfactory effects can be obtained when the compound of the present invention or its crystalline form is administered at a dose of about 0.25-1000 mg/kg of animal body weight per day, preferably administered in divided doses 2-4 times per day, or administered in sustained release form. This dosage regimen can be adjusted to provide the optimum therapeutic response. In addition, several divided doses may be administered daily or the dose may be proportionally reduced, depending on the therapeutic situation.

The compound or its crystalline form according to the present invention and the pharmaceutical composition of the present invention can be used for the relevant conditions suitable for preventing and/or treating various diseases and diseases, in particular disorders characterized by elevated plasma aldosterone concentrations or changes in plasma aldosterone concentrations relative to plasma renin concentrations, or conditions associated with these changes. Examples that may be mentioned are: spontaneous primary aldosteronism, hyperaldosteronism associated with adrenal hyperplasia, adrenal adenoma and/or adrenal carcinoma, hyperaldosteronism associated with cirrhosis, hyperaldosteronism associated with heart failure, and (relative) hyperaldosteronism associated with essential hypertension, *etc.*

Specifically, the compound or its crystalline form involved in the present invention can be used to treat or prevent the following diseases or conditions: diabetic nephropathy, hyperaldosteronism, hypertension, heart failure, sequelae of myocardial infarction, liver cirrhosis, nonalcoholic steatohepatitis, chronic kidney disease, fibrosis, renal failure, or stroke in a subject.

The X-ray powder diffraction analysis method used in the present invention was an Empyrean diffractometer, and an X-ray powder diffraction pattern was obtained using Cu-Kα radiation (45 KV, 40 mA). The powdery sample was prepared as a thin layer on a monocrystalline silicon sample rack and placed on a rotating sample stage, analyzed at a rate of 0.0167° steps in the range of 3°-60°. Data Collector software was used to collect data, HighScore Plus software was used to process data, and Data Viewer software was used to read data.

The differential scanning calorimetry (DSC) analysis method used in the present invention is a differential scanning calorimeter using a TA Q2000 module with a thermal analysis controller. Data were collected and analyzed using TA Instruments Thermal Solutions software. Approximately 1-5 mg of the sample was accurately weighed into a specially crafted aluminum crucible with a lid and analyzed from room temperature to about 300 °C using a linear heating device at 10 °C/min. During use, the DSC chamber was purged with dry nitrogen.

The thermogravimetric (TGA) analysis method used in the present invention is to perform thermogravimetric loss using a TA Q500 module with a thermal analysis controller. Data were collected and analyzed using TA Instruments Thermal Solutions software. Approximately 10 mg of the sample was accurately weighed into a platinum sample pan and analyzed from room temperature to about 300°C using a linear heating device at 10°C/min. During use, the TGA furnace chamber was purged with dry nitrogen.

The solubility of the present invention was determined using an Agilent 1200 High Performance Liquid Chromatograph DAD/VWD detector with an Agilent XDB-C18 model (4.6 x 50 mm, 5 µm). Detection wavelength was 266 nm, flow rate was 1.0 mL/min, column temperature was 35 °C, mobile phase A: acetonitrile-0.01M ammonium acetate=10:90 (V:V), analysis method: acetonitrile-mobile phase A=70 : 30 (V:V), run time: 10 minutes.

The hygroscopicity of the present invention was measured by a DVS INT-Std dynamic moisture and gas adsorption analyzer from Surface Measurement Systems, UK. The humidity test range: 0%-95%, air flow: 200 mL/min, temperature: 25 °C, test point: every 5% increase in humidity to take a test point.

### SPECIFIC PREPARATION METHOD

The present invention provides preparation examples of the compound having formula (I) and its crystalline form. Skilled in the art can learn from this article to properly improve the process parameters to implement the preparation method. Related person can clearly realize and apply the techniques disclosed herein by making some changes, appropriate alterations or combinations to the methods without departing from the scope of the present disclosure.

In order to further understand the invention, it is detailed below through examples.

### Example

### Example 1 Amorphous form of the compound having formula (I) ((S)-1-(2-hydroxyethyl)-4-methyl-N-(3-fluoro-4-(methylsulfonyl)phenyl)-5-(2-(trifluoromethyl)phenyl)-1H-pyrrole-3-carboxamide)

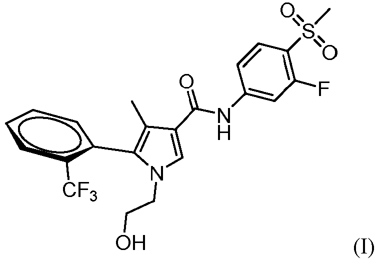

**The title compound was obtained by referring to the preparation methods of Examples 1 and 2 in** WO2021078135A1**. The detailed process is as follows:**

### Step 1) Ethyl 5-bromo-4-methyl-1H-pyrrole-3-carboxylate

To a 100 ml flask were added ethyl 4-methylpyrrole-3-carboxylate (6.0 g, 39 mmol) and tetrahydrofuran (50 mL). The mixture was cooled to -78°C, then N-bromosuccinimide (6.99 g, 39.3 mmol) was added, and the mixture was stirred at -78°C for 15 min, then 6 drops of pyridine was added. The mixture was slowly raised to 5°C, and continued stirring overnight. The reaction solution was extracted with ethyl acetate (100 mL × 3), dried over anhydrous sodium sulfate, filtered, and concentrated *in vacuo,* and the residue was separated by silica gel column chromatography (petroleum ether/ethyl acetate (v/v) = 10/1) to obtain a white solid (7.41 g, 82%).

MS (ESI, pos. ion) m/z: 232.1 (M+1).

¹H NMR (400 MHz, DMSO-*d*₆) δ (ppm) 11.97 (s, 1H), 7.40 (s, 1H), 4.15 (q, *J* = 7.1 Hz, 2H), 2.11 (s, 3H), 1.24 (t, *J =* 7.1 Hz, 3H).

### Step 2) Ethyl 4-methyl-5-(2-(trifluoromethyl)phenyl)-1H-pyrrole-3-carboxylate

To a 500 ml flask were added ethyl 5-bromo-4-methyl-1H-pyrrole-3-carboxylate (15.8 g, 68.1 mmol), 2-(trifluoromethyl)phenylboronic acid (20.9 g, 110 mmol), lithium chloride (289.3 mg, 6.83 mmol), sodium carbonate solution (68 mL, 136 mmol, 2 mol/L), 1,4-dioxane (200 mL) and [1,1'-bis(diphenylphosphino)ferrocene]dichloropalladium dichloromethane complex (3.49 g, 4.19 mmol). The mixture was heated to 90°C and reacted for 22 h after the addition was complete. The solvent was removed by rotary evaporation under reduced pressure. The resulting mixture was extracted with ethyl acetate (80 mL × 4), dried over anhydrous sodium sulfate, filtered, and concentrated *in vacuo,* and the residue was separated by silica gel column chromatography (petroleum ether/dichloromethane (v/v) = 5/1) to obtain a white solid (6.9 g, 34%).

MS (ESI, pos. ion) m/z: 298.2 (M+1).

### Step 3) Ethyl 1-(2-(benzyloxy)ethyl)-4-methyl-5-(2-(trifluoromethyl)phenyl)-1H-pyrrole-3-carboxylate

To a 500 ml flask were added ethyl 4-methyl-5-(2-(trifluoromethyl)phenyl)-1*H*-pyrrole-3-carboxylate (25 g, 84.1 mmol), *N,N*-dimethylformamide (160 mL), cesium carbonate (41.7 g, 128 mmol) and benzyl 2-bromoethyl ether (16 mL, 101 mmol), The mixture was heated to 70°C and stirred for 12 h after the addition was complete. The reaction solution was extracted with ethyl acetate (150 mL × 3). The organic phases were combined and washed with saturated brine (150 mL × 3), dried over anhydrous sodium sulfate, filtered, and concentrated *in vacuo,* and the residue was separated by silica gel column chromatography (petroleum ether/ethyl acetate (v/v) = 10/1) to obtain a tan solid (36.2 g, 99.8%).

MS (ESI, pos. ion) m/z: 432.4 (M+1).

### Step 4) 1-(2-(Benzyloxy)ethyl)-4-methyl-5-(2-(trifluoromethyl)phenyl)-1H-pyrrole-3-carboxylic acid

To a 1000 ml flask were added ethyl 1-(2-(benzyloxy)ethyl)-4-methyl-5-(2-(trifluoromethyl)phenyl)-1*H*-pyrrole-3-carboxylate (36.2 g, 83.9 mmol), sodium hydroxide solution (60 mL, 960 mmol, 16 mol/L) and ethanol (200 mL). The mixture was heated to 70°C and stirred overnight after the addition was complete. Ethanol was removed by rotary evaporation under reduced pressure, water (1000 mL) was added to the residue and the resulting mixture was stirred at room temperature for 30 min, then washed with methyl *tert*-butyl ether (200 mL × 3). The aqueous phase was adjusted to pH = 2 with 6 M HCl solution, extracted with ethyl acetate (200 mL × 3), dried over anhydrous sodium sulfate, filtered, and concentrated *in vacuo* to give a tan solid (33.4 g, 98.7%).

MS (ESI, pos. ion) m/z: 404.2 (M+1).

### Step 5) 1-(2-(Benzyloxy)ethyl)-4-methyl-5-(2-(trifluoromethyl)phenyl)-1H-pyrrole-3-carbonyl chloride

To a 100 ml flask were added 1-(2-(benzyloxy)ethyl)-4-methyl-5-(2-(trifluoromethyl)phenyl)-1*H*-pyrrole-3-carboxylic acid (401 mg, 0.994 mmol), dichloromethane (15 mL) and 2 drops of N,N-dimethylformamide. Oxalyl chloride (0.40 mL, 4.7 mmol) was added dropwise under ice-cooling conditions. The mixture was moved to room temperature and reacted for 2.5 h after the addition was complete. The solvent was removed by rotary evaporation under reduced pressure to give a tan solid (400 mg, 95.40%).

### Step 6) 1-(2-(Benzyloxy)ethyl)-N-(3-fluoro-4-(methylsulfonyl)phenyl)-4-methyl-5-(2-(trifluoromethyl) )phenyl)-1H-pyrrole-3-carboxamide

To a 50 mL sealed tube were added 1-(2-(benzyloxy)ethyl)-4-methyl-5-(2-(trifluoromethyl)phenyl)-1H-pyrrole-3-carbonyl chloride (400 mg, 0.948 mmol), tetrahydrofuran (10 mL), pyridine (0.14 mL, 1.7 mmol), 3-fluoro-4-thiamphenicol aniline (160 mg, 0.846 mmol) and 4-dimethylaminopyridine (10.7 mg, 0.0876 mmol). The mixture was heated to 80°C and stirred overnight after the addition was complete. The solvent was evaporated under reduced pressure. The residue was purified by silica gel column chromatography (petroleum ether / ethyl acetate = 2/1) to give a light yellow solid (337 mg, 69.36%).

MS (ESI, pos. ion) m/z: 575.2 (M+1).

### Step 7) N-(3-fluoro-4-(methylsulfonyl)phenyl)-1-(2-hydroxyethyl)-4-methyl-5-(2-(trifluoromethyl)phenyl) -1H-pyrrole-3-carboxamide

To a 100 ml flask were added 1-(2-(benzyloxy)ethyl)-*N*-(3-fluoro-4-(methylsulfonyl)phenyl)-4-methyl-5-(2-(trifluoromethyl) phenyl)-1*H*-pyrrole-3-carboxamide (337 mg, 0.587 mmol), methanol (10 mL) and palladium on carbon (66.7 mg, 10 mass%). After the addition was complete, the mixture was stirred at room temperature under hydrogen atmosphere for 1.5 h. The reaction solution was suction filtered through a celite pad, the filter cake was washed with methanol (10 mL × 3), the filtrate was concentrated, and the residue was separated by silica gel column chromatography (petroleum ether/ethyl acetate (v/v) = 1/2) to obtain a white solid (220.5 mg, 77.60%).

MS (ESI, pos. ion) m/z: 485.3 (M+1).

### Step 8) (S)-1-(2-Hydroxyethyl)-4-methyl-N-(3-fluoro-4-(methylsulfonyl)phenyl)-5-(2-(trifluoromethyl) )phenyl)-1H-pyrrole-3-carboxamide

To a 25 ml flask was added *N*-3-fluoro-4-(methylsulfonyl)phenyl)-1-(2-hydroxyethyl)-4-methyl-5-(2-(trifluoromethyl)phenyl)-1*H*-pyrrole -3-carboxamide (3.4 g, 7.02 mmol), and anhydrous acetonitrile (8 mL) was added to dissolve the sample completely. Chiral resolution was performed by high performance liquid chromatography (instrument: waters SFC; chromatographic column: daicel AS-H 10 mm × 250 mm 5 µm; condition: isocratic 20% MeOH + 80% CO₂; flow rate: 8 mL/min; column temperature: 35°C; back pressure: 100 bar; 10 µL per injection), and the solvent was removed by rotary evaporation under reduced pressure to obtain the title compound as a white solid (1.43 g, 42.1%).
MS (ESI, pos. ion) m/z: 485.1 (M+1);
¹H NMR (400 MHz, DMSO-*d*₆) δ (ppm): 10.15 (s, 1H), 7.98 (dd, *J =* 13.5, 1.3 Hz, 1H), 7.90 (d, *J =* 7.8 Hz, 1H), 7.82 - 7.76 (m, 3H), 7.75 - 7.68 (m, 2H), 7.47 (d, *J =* 7.4 Hz, 1H), 4.94 (t, *J* = 4.9 Hz, 1H), 3.73 - 3.64 (m, 1H), 3.56 - 3.45 (m, 3H), 3.28 (s, 3H), 1.92 (s, 3H).

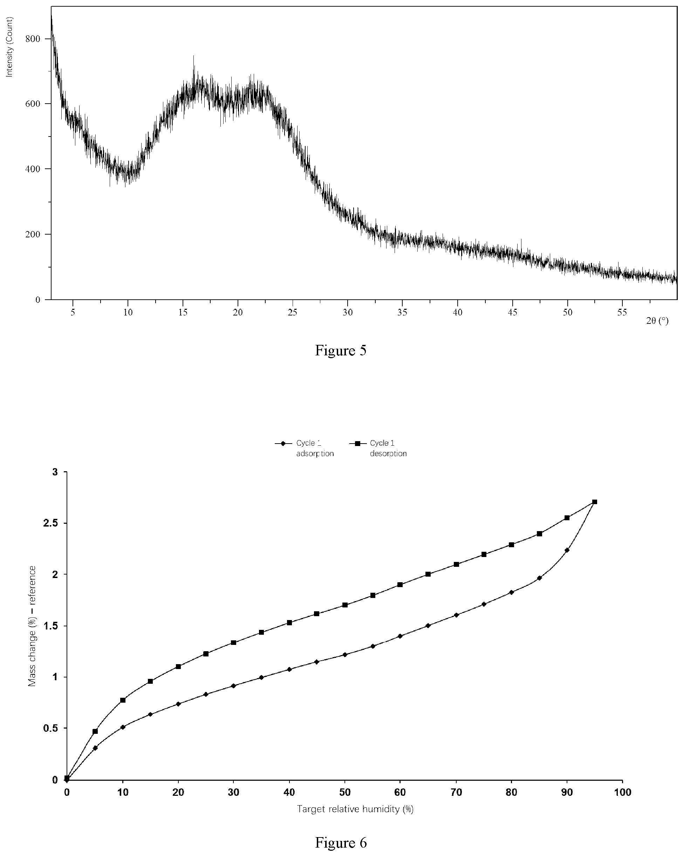

Analysis and identification by Empyrean X-ray powder diffraction (XRPD): the white solid is amorphous, and its XRPD spectrum is basically as shown in Figure 5.

### Example 2 Crystalline form I of the compound having Formula (I)

### 1. Preparation of crystalline form I

Method 1: the compound of Example 1 (that is, the amorphous form of the compound having formula (I)) (30.0 g) was added to isopropyl acetate (120.0 mL). The mixture was heated to 60°C to dissolve the solid, then n-heptane (90.0 mL) was slowly added dropwise. The temperature was kept and the mixture was stirred until the solid precipitated, then the heating was turned off, and the reaction mixture was naturally cooled to room temperature; the mixture was filtered, and the filter cake was washed with a mixed solvent (30.0 mL × 2) of isopropyl acetate and n-heptane (v/v = 1/2) and dried under vacuum at 60°C overnight to obtain a white solid (25.63 g, 85.43%).

Method 2: the compound of Example 1 (10.0 g) was added to isopropyl acetate (30.0 mL). The mixture was heated to 55°C to obtain a clear solution, then toluene (150.0 mL) was added dropwise. The temperature was kept and the mixture was stirred until the solid precipitated, then the heating was turned off, and the mixture was naturally cooled to room temperature and stirred overnight; the mixture was filtered, and the filter cake was washed with toluene (10.0 mL × 2) and dried under vacuum at 60°C for 8.0 hours to obtain a white solid (8.17 g, 81.7%).

Method 3: the compound of Example 1 (1.0 g) was added to ethanol (5.0 mL). The mixture was stirred at room temperature to dissolve the solid, then water (10 mL) was slowly added dropwise. The temperature was kept and the mixture stirred until the solid precipitated; then the mixture was filtered, and the filter cake was washed with water (1.0 mL × 2) and dried under vacuum at 60°C overnight to obtain a white solid (934 mg, 93.4%).

### 2. Identification of crystalline form I

(1) Analysis and identification by Empyrean X-ray powder diffraction (XRPD): using Cu-Kα radiation, the white solid prepared above has the following diffraction peaks expressed in 2θ angles: 6.89°, 10.71°, 11.31°, 12.25°, 13.58°, 13.70°, 13.99°, 14.45°, 15.19°, 16.42°, 17.04°, 18.00°, 18.31°, 19.35°, 19.97°, 20.32°, 20.65°, 21.48°, 21.83°, 21.95°, 22.25°, 22.51°, 23.06°, 23.16°, 23.31°, 24.59°, 24.78°, 24.93°, 25.77°, 26.22°, 26.98°, 27.46°, 27.75°, 28.15°, 29.11°, 29.33°, 29.82°, 30.51°, 30.83°, 31.44°, 31.96°, 32.42°, 32.87°, 33.74°, 34.77°, 35.87°, 36.25°, 36.52°, 37.33°, 37.98°, 38.43°, 39.08°, 39.34°, 40.16°, 40.78°, 41.47°, 41.94°, 42.18°, 42.71°, 43.61°, 44.35°, 44.66°, 45.05°, 45.39°, 45.88°, 46.69°, 47.21°, 47.66°, 48.32°, 48.97°, 49.38°, 50.35°, 50.65°, 51.97°, 53.24°, 55.00°, 56.23° and 58.12°. There is an error tolerance of ± 0.2°.

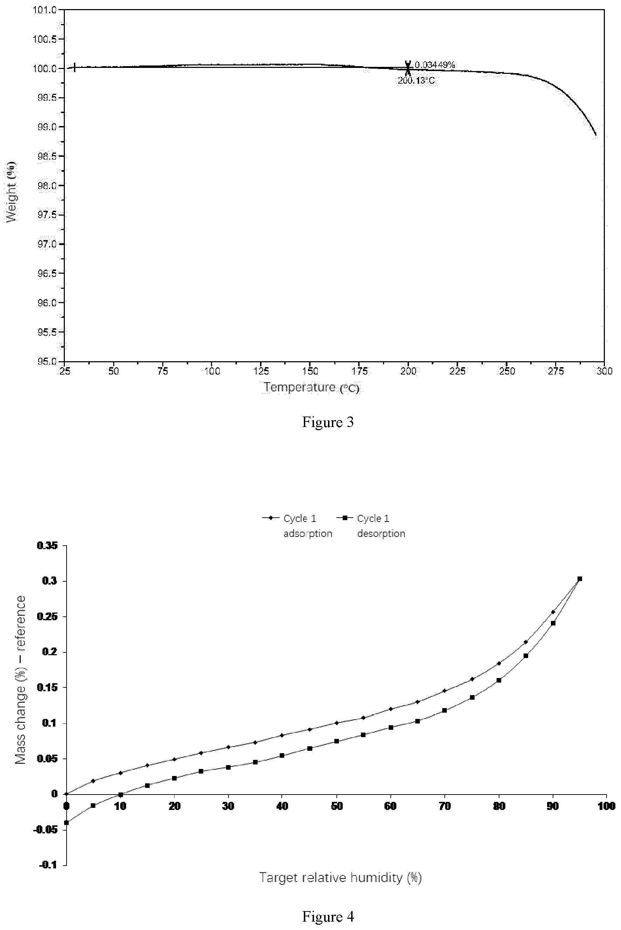

Specifically, the crystalline form I of the present invention has an X-ray powder diffraction pattern substantially as shown in Figure 1.
(2) Analysis and identification by TA Q2000 Differential Scanning Calorimetry (DSC): the scanning speed was 10 °C/min and it contained an endothermic peak of 158.49 °C. There is an error tolerance of ± 3 °C. Specifically, the crystalline form I of the present invention has a differential scanning calorimetry diagram substantially as shown in Figure 2.
(3) Thermal gravimetric analysis (TGA) and identification by TA Q500: the heating rate was 10°C/min, the weight loss range was 0.03%, and there is an error tolerance of ± 0.1%. Specifically, the crystalline form I of the present invention has a thermal gravimetric analysis (TGA) diagram substantially as shown in Figure 3.

### Example 3 Pharmacokinetic experiment

The crystalline form I prepared by referring to any method of Example 2 was filled into capsules, which are used for oral administration.

8-12 kg Male Beagle dogs were divided into 2 groups, and 3 in each group. Each Beagle dog was orally administered the capsule containing the test sample at a dose of 5 mg/kg, and blood was collected at time points of 0.25, 0.5, 1.0, 2.0, 4.0, 6.0, 8.0 and 24 hours. Standard curve was plotted based on concentrations of the samples in a suitable range, the concentration of the test sample in the plasma sample was measured and quantified by AB SCIEX API4000 LC-MS / MS at MRM mode. Pharmacokinetic parameters were calculated according to drug concentration-time curve using a noncompartmental method by WinNonLin 6.3 software. Results are as shown in table 1.

A pharmacokinetic experiment of the amorphous form prepared with reference to Example 1 was carried out according to the above method, and the results are shown in Table 1.

**Table 1 Pharmacokinetic experimental data of the crystalline form of the present invention**

| Test sample | AUCₗₐₛₜ (h*ng/ml) | Cₘₐₓ (ng/ml) | Tₘₐₓ (h) |
|---|---|---|---|
| Crystalline form I | 42300 | 1450 | 4.0 |
| Amorphous form | 20900 | 913 | 3.33 |

### Conclusion:

As can be seen from Table 1, the crystalline form I of the present invention has good pharmacokinetic properties. Specifically, the crystalline form I of the present invention has higher exposure and blood drug concentration than the amorphous form in Beagle dogs, and has better pharmacokinetic properties.

### Example 4 Stability experiment

Test samples: crystalline form I was prepared by referring to any method of Example 2, and amorphous form was prepared by referring to Example 1.

The following tests of test samples were carried out:
(1) High temperature test: an appropriate amount of the test sample was taken into a flat weighing bottle, spread into a thin layer with a thickness of ≤ 5 mm. The samples were placed at 60 °C ± 2 °C for 30 days. The samples were taken for testing according to the key stability inspection items on the 5th, 10th, and 30th day. The color change of the samples was observed. The purity of the samples was determined by HPLC. Results are as shown in Table 2.

**Table 2 Results of high temperature tests**

| Test sample | | Crystalline form I | Amorphous form |
|---|---|---|---|
| Appearance | 0 day | White | Off-white |
| | 5 days | White | Off-white |
| | 10 days | White | Off-white |
| | 30 days | White | Off-white |
| Total impurity % | 0 day | 0.34 | 0.40 |
| | 5 days | 0.36 | 0.46 |
| | 10 days | 0.34 | 0.55 |
| | 30 days | 0.34 | 1.28 |

(2) High humidity test: an appropriate amount of the test sample was taken into a flat weighing bottle, spread into a thin layer with a thickness of ≤ 5 mm. The samples were placed at 25 °C and RH for 90% ± 5% for 30 days. The samples were taken for testing according to the key stability inspection items on the 5th, 10th, and 30th day. The color change of the samples was observed. The purity of the samples was determined by HPLC. Results are as shown in Table 3.

**Table 3 Results of high humidity tests**

| Test sample | | Crystalline form I | Amorphous form |
|---|---|---|---|
| Appearance | 0 day | White | Off-white |
| | 5 days | White | Off-white |
| | 10 days | White | Off-white |
| | 30 days | White | Off-white |
| Total impurity % | 0 day | 0.34 | 0.40 |
| | 5 days | 0.35 | 0.47 |
| | 10 days | 0.36 | 0.53 |
| | 30 days | 0.34 | 0.57 |

(3) Light test: an appropriate amount of the test sample was taken into a flat weighing bottle, spread into a thin layer with a thickness of ≤ 5 mm. The samples were placed in a light box (with a UV lamp) and placed under the conditions of 25°C, white light 4990 lux, and UV 1.4 W·h/m² for 30 days. The samples were taken for testing on the 5th, 10th, and 30th day. The color change of the samples was observed. The purity of the samples was determined by HPLC. Results are as shown in Table 4.

**Table 4 Results of light tests**

| Test sample | | Crystalline form I | Amorphous form |
|---|---|---|---|
| Appearance | 0 day | White | Off-white |
| | 5 days | White | Surface turned yellow |
| | 10 days | White | Surface turned yellow |
| | 30 days | White | Surface turned yellow |
| Total impurity % | 0 day | 0.34 | 0.40 |
| | 5 days | 0.34 | 0.85 |
| | 10 days | 0.32 | 1.79 |
| | 30 days | 0.49 | 3.47 |

(4) Accelerated test: an appropriate amount of crystalline form I test samples in a single-layer PE package plus aluminum foil packaging was stored for 6 months under the conditions of 40 ± 2 °C/75% ± 5% RH. Samples were taken for testing in the 1st, 2nd, 3rd and 6th month. The color change of the samples was observed. The purity of the samples was determined by HPLC. Results are as shown in Table 5.

**Table 5 Results of acceleration tests**

| Test sample | | Crystalline form I |
|---|---|---|
| Appearance | 0 day | White |
| | 1 month | White |
| | 2 months | White |
| | 3 months | White |
| | 6 months | White |
| Total impurity % | 0 day | 0.78 |
| | 1 month | 0.72 |
| | 2 months | 0.73 |
| | 3 months | 0.73 |
| | 6 months | 0.71 |

### Conclusion:

It can be seen from the experimental results that the crystalline form I described in the present invention has better stability than the amorphous form. Under the conditions of high temperature (60°C), high humidity (25°C, RH 90% ± 5%) and light, the appearance and purity of the crystalline form I described in the present invention have no obvious changes, and the form has good stability. Furthermore, under the accelerated test conditions, the appearance and purity of the crystalline form I in the present invention do not change significantly.

In summary, the crystalline form I described in the present invention has good stability under various setting-out conditions and is suitable for pharmaceutical use.

### Example 5 Hygroscopicity experiment

Test samples: crystalline form I prepared by referring to any method of Example 2, and amorphous form prepared by referring to Example 1.

An appropriate amount of the test sample was taken, and the hygroscopicity was tested by a dynamic moisture adsorption device. The experimental results are shown in Table 7. The description of the hygroscopicity feature and the definition of the hygroscopicity gain (Chinese Pharmacopoeia 2020 edition, Appendix 9103 Guidelines for drug dampness test, experimental conditions: 25 °C ± 1 °C, 80% ± 2% relative humidity) as described in Table 6 below:

**Table 6 The description of the hygroscopicity feature and the definition of the hygroscopicity gain**

| The hygroscopicity feature | The hygroscopicity gain |
|---|---|
| Deliquescence | Absorb enough water to form a liquid |
| Highly hygroscopic | Not less than 15% |
| Hygroscopicity | Less than 15% but not less than 0.2% |
| Slightly hygroscopic | Less than 2% but not less than 0.2% |
| No or almost none hygroscopicity | Less than 0.2% |

From the experimental results in Table 7 below, it can be seen that the crystalline form I of the present invention has no or almost no hygroscopicity, and is not easy to deliquescence under the influence of high humidity; the amorphous form of the present invention has a slight hygroscopicity. The DVS diagrams of the hygroscopicity experiments of the crystalline form I and the amorphous form of the present invention are basically shown in Figure 4 and Figure 6.

**Table 7 Results of hygroscopicity experiment**

| Test sample | Weight gain at 60% relative humidity/% | Weight gain at 80% relative humidity/% | Weight gain at 95% relative humidity/% |
|---|---|---|---|
| Crystalline form I | 0.120 | 0.185 | 0.304 |
| Amorphous form | 1.401 | 1.826 | 2.707 |

In summary, compared with the amorphous form, the crystalline form I of the compound having formula (I) of the present invention has obvious advantages in terms of pharmacokinetic properties, stability, and hygroscopicity, and has better druggability.

Reference throughout this specification to "an embodiment", "some embodiments", "one embodiment", "another example", "an example", "a specific example", or "some examples" means that a particular feature, structure, material, or characteristic described in connection with the embodiment or example is included in at least one embodiment or example of the present disclosure. In this specification, the schematic representations of the above terms are not necessarily directed to the same embodiment or example. Furthermore, the particular features, structures, materials, or characteristics may be combined in any suitable manner in one or more embodiments or examples. In addition, those skilled in the art can integrate and combine different embodiments, examples or the features of them as long as they are not contradictory to one another.

## Claims

1. A crystalline form I of the compound having Formula (I): 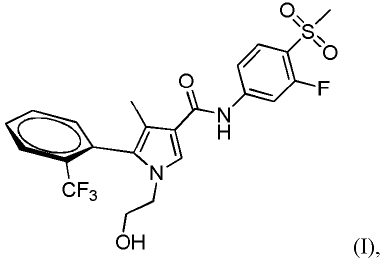 **characterized in that** the X-ray powder diffraction pattern of the crystalline form I has diffraction peaks at the following 2θ angles: 14.45°± 0.2°, 17.04°± 0.2°, 19.35°± 0.2°, 22.51°± 0.2°, 24.78°± 0.2° wherein the X-ray powder diffraction pattern was obtained using Cu-Kα radiation.

2. The crystalline form I of claim 1, **characterized in that** the X-ray powder diffraction pattern of the crystalline form I has diffraction peaks at the following 2θ angles: 6.89°±0.2°, 14.45°±0.2°, 16.42°± 0.2°, 17.04°± 0.2°, 18.31°± 0.2°, 19.35°± 0.2°, 22.25°± 0.2°, 22.51°± 0.2°, 23.06°± 0.2°, 24.78°± 0.2°.

3. The crystalline form I of claim 1 or 2, **characterized in that** the X-ray powder diffraction pattern of the crystalline form I also has diffraction peaks at at least one of the following 2θ angles: 12.25°±0.2°, 13.70°±0.2°, 13.99°± 0.2°, 20.65°± 0.2°, 21.83°± 0.2°, 21.95°± 0.2°, 24.59°± 0.2°, 24.93°± 0.2°, 25.77°± 0.2°, 26.98°± 0.2°.

4. The crystalline form I of any one of claims 1-3, **characterized in that** the X-ray powder diffraction pattern of the crystalline form I also has diffraction peaks at at least one of the following 2θ angles: 13.58°±0.2°, 15.19°±0.2°, 18.00°± 0.2°, 19.97°± 0.2°, 23.16°± 0.2°, 29.11°± 0.2°, 29.33°± 0.2°, 31.96°± 0.2°, 32.42°± 0.2°, 33.74°± 0.2°.

5. The crystalline form I of any one of claims 1-4, **characterized in that** the X-ray powder diffraction pattern of the crystalline form I also has diffraction peaks at at least one of the following 2θ angles: 10.71°± 0.2°, 11.31°± 0.2°, 20.32°± 0.2°, 21.48°± 0.2°, 23.31°± 0.2°, 27.46°± 0.2°, 27.75°± 0.2°, 28.15°± 0.2°, 29.82°± 0.2°, 30.51°± 0.2°, 30.83°± 0.2°, 32.87°± 0.2°, 34.78°± 0.2°, 35.87°± 0.2°, 36.25°± 0.2°, 36.52°± 0.2°, 37.33°± 0.2°, 37.98°± 0.2°, 38.43°± 0.2°, 39.08°± 0.2°, 39.34°± 0.2°, 40.16°± 0.2°, 40.78°± 0.2°, 41.94°± 0.2°, 42.18°± 0.2°, 43.61°± 0.2°, 44.35°± 0.2°, 44.66°± 0.2°, 45.39°± 0.2°, 47.66°± 0.2°.

6. The crystalline form I of any one of claims 1-4, **characterized in that** the X-ray powder diffraction pattern of the crystalline form I has diffraction peaks at the following 2θ angles: 6.89°± 0.2°, 10.71°± 0.2°, 12.25°± 0.2°, 13.58°± 0.2°, 13.70°± 0.2°, 13.99°± 0.2°, 14.45°± 0.2°, 15.19°± 0.2°, 16.42°± 0.2°, 17.04°± 0.2°, 18.00°± 0.2°, 18.31°± 0.2°, 19.35°± 0.2°, 19.97°± 0.2°, 20.32°± 0.2°, 20.65°± 0.2°, 21.83°± 0.2°, 21.95°± 0.2°, 22.25°± 0.2°, 22.51°± 0.2°, 23.06°± 0.2°, 23.16°± 0.2°, 23.31°± 0.2°, 24.59°± 0.2°, 24.78°± 0.2°, 24.93°± 0.2°, 25.77°± 0.2°, 26.98°± 0.2°, 27.46°± 0.2°, 27.75°± 0.2°, 29.11°± 0.2°, 29.33°± 0.2°, 30.51°± 0.2°, 30.83°± 0.2°, 31.96°± 0.2°, 32.42°± 0.2°, 33.74°± 0.2°, 35.87°± 0.2°, 36.25°± 0.2°, 38.43°± 0.2°, 40.16°± 0.2°, 41.94°± 0.2°, 42.18°± 0.2°, 43.61°± 0.2°.

7. The crystalline form I of any one of claims 1-4, **characterized in that** the X-ray powder diffraction pattern of the crystalline form I has diffraction peaks at the following 2θ angles: 6.89°± 0.2°, 10.71°± 0.2°, 11.31°± 0.2°, 12.25°± 0.2°, 13.58°± 0.2°, 13.70°± 0.2°, 13.99°± 0.2°, 14.45°± 0.2°, 15.19°± 0.2°, 16.42°± 0.2°, 17.04°± 0.2°, 18.00°± 0.2°, 18.31°± 0.2°, 19.35°± 0.2°, 19.97°± 0.2°, 20.32°± 0.2°, 20.65°± 0.2°, 21.48°± 0.2°, 21.83°± 0.2°, 21.95°± 0.2°, 22.25°± 0.2°, 22.51°± 0.2°, 23.06°± 0.2°, 23.16°± 0.2°, 23.31°± 0.2°, 24.59°± 0.2°, 24.78°± 0.2°, 24.93°± 0.2°, 25.77°± 0.2°, 26.98°± 0.2°, 27.46°± 0.2°, 27.75°± 0.2°, 28.15°± 0.2°, 29.11°± 0.2°, 29.33°± 0.2°, 29.82°± 0.2°, 30.51°± 0.2°, 30.83°± 0.2°, 31.96°± 0.2°, 32.42°± 0.2°, 32.87°± 0.2°, 33.74°± 0.2°, 34.77°± 0.2°, 35.87°± 0.2°, 36.25°± 0.2°, 36.52°± 0.2°, 37.33°± 0.2°, 37.98°± 0.2°, 38.43°± 0.2°, 39.08°± 0.2°, 39.34°± 0.2°, 40.16°± 0.2°, 40.78°± 0.2°, 41.94°± 0.2°, 42.18°± 0.2°, 43.61°± 0.2°, 44.35°± 0.2°, 44.66°± 0.2°, 45.39°± 0.2°, 47.66°± 0.2°.

8. The crystalline form I of any one of claims 1-4, **characterized in that** the X-ray powder diffraction pattern of the crystalline form I has diffraction peaks at the following 2θ angles: 6.89°± 0.2°, 10.71°± 0.2°, 11.31°± 0.2°, 12.25°± 0.2°, 13.58°± 0.2°, 13.70°± 0.2°, 13.99°± 0.2°, 14.45°± 0.2°, 15.19°± 0.2°, 16.42°± 0.2°, 17.04°± 0.2°, 18.00°± 0.2°, 18.31°± 0.2°, 19.35°± 0.2°, 19.97°± 0.2°, 20.32°± 0.2°, 20.65°± 0.2°, 21.48°± 0.2°, 21.83°± 0.2°, 21.95°± 0.2°, 22.25°± 0.2°, 22.51°± 0.2°, 23.06°± 0.2°, 23.16°± 0.2°, 23.31°± 0.2°, 24.59°± 0.2°, 24.78°± 0.2°, 24.93°± 0.2°, 25.77°± 0.2°, 26.22°± 0.2°, 26.98°± 0.2°, 27.46°± 0.2°, 27.75°± 0.2°, 28.15°± 0.2°, 29.11°± 0.2°, 29.33°± 0.2°, 29.82°± 0.2°, 30.51°± 0.2°, 30.83°± 0.2°, 31.44°± 0.2°, 31.96°± 0.2°, 32.42°± 0.2°, 32.87°± 0.2°, 33.74°± 0.2°, 34.77°± 0.2°, 35.87°± 0.2°, 36.25°± 0.2°, 36.52°± 0.2°, 37.33°± 0.2°, 37.98°± 0.2°, 38.43°± 0.2°, 39.08°± 0.2°, 39.34°± 0.2°, 40.16°± 0.2°, 40.78°± 0.2°, 41.47°± 0.2°, 41.94°± 0.2°, 42.18°± 0.2°, 42.71°± 0.2°, 43.61°± 0.2°, 44.35°± 0.2°, 44.66°± 0.2°, 45.05°± 0.2°, 45.39°± 0.2°, 45.88°± 0.2°, 46.69°± 0.2°, 47.21°± 0.2°, 47.66°± 0.2°, 48.32°± 0.2°, 48.97°± 0.2°, 49.38°± 0.2°, 50.35°± 0.2°, 50.65°± 0.2°, 51.97°± 0.2°, 53.24°± 0.2°, 55.00°± 0.2°, 56.23°± 0.2°, 58.12°± 0.2°.

9. The crystalline form I of any one of claims 1-7, **characterized in that** the differential scanning calorimetry diagram of the crystalline form I comprises an endothermic peak at 158.49°C ± 3°C, wherein the differential scanning calorimetry diagram was obtained from room temperature to about 300°C using a linear heating device at 10°C/min.

10. A method for preparing the crystalline form I of the compound having Formula (I) of any one of claims 1-9, comprising:
step 1): the compound having Formula (I) is added into solvent 1, and the resulting suspension is stirred until dissolved;
step 2): solvent 2 is added dropwise to the clear solution in step 1); and
step 3): the resulting mixture is stirred and crystallized.

11. The method of claim 10, **characterized in that** the solvent 1 is ethyl acetate, n-propyl acetate, isopropyl acetate, *tert*-butyl acetate, methyl acetate, methanol, ethanol or isopropanol.

12. The method of claim 10 or 11, **characterized in that** the solvent 2 is n-hexane, cyclohexane, n-heptane, xylene, toluene or water.

13. The method of any one of claims 10-12, **characterized in that** the solvent 1 is isopropyl acetate, and the solvent 2 is n-heptane or toluene; or, the solvent 1 is ethanol, and the solvent 2 is water.

14. The method of any one of claims 10-13, **characterized in that** in the step 1), the suspension is stirred at room temperature or heated to a certain temperature until clear, wherein the certain temperature is 40°C-80°C.

15. The method of any one of claims 10-14, **characterized in that** in the step 3), the stirring and crystallization is carried out at room temperature or at a certain temperature, wherein the certain temperature is 40°C-80°C.

16. A pharmaceutical composition, comprising the crystalline form I of any one of claims 1-9, and a pharmaceutically acceptable carrier, excipient, diluent, adjuvant or a combination thereof.

17. The pharmaceutical composition of claim 16, further comprising one or more other active ingredients selected from SGLT-2 inhibitors, sGC activators, sGC stimulators, ACE inhibitors, renin inhibitors, angiotensin II receptor antagonists, β-blockers, acetylsalicylic acid, diuretics, calcium antagonists agents, statins, digitalis derivatives, calcium sensitizers, nitrates and antithrombotics.

18. The crystalline form I of any one of claims 1-9 or the pharmaceutical composition of any one of claims 16-17 for use in treating, preventing or alleviating the following disease in patients: diabetic nephropathy, hyperaldosteronism, hypertension, heart failure, sequelae of myocardial infarction, liver cirrhosis, nonalcoholic steatohepatitis, chronic kidney disease, fibrosis, renal failure, or stroke.

## Patentansprüche

1. Kristalline Form I der Verbindung mit Formel (I): 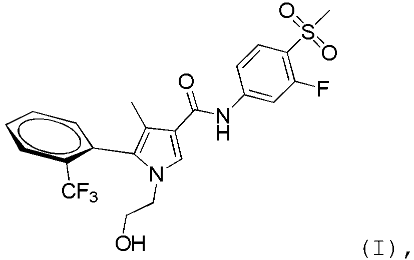 **dadurch gekennzeichnet, dass** das Röntgenpulverbeugungsmuster der kristallinen Form I Beugungspeaks bei den folgenden 2θ-Winkeln aufweist: 14,45°±0,2°, 17,04°±0,2°, 19,35°±0,2°, 22,51°±0,2°, 24,78°±0,2°, wobei das Röntgenpulverbeugungsmuster unter Verwendung von Cu-Kα-Strahlung erhalten wurde.

2. Kristalline Form I nach Anspruch 1, **dadurch gekennzeichnet, dass** das Röntgenpulverbeugungsmuster der kristallinen Form I Beugungspeaks bei den folgenden 2θ-Winkeln aufweist: 6,89°±0,2°, 14,45°±0,2°, 16,42°±0,2°, 17,04°±0,2°, 18,31°±0,2°, 19,35°±0,2°, 22,25°±0,2°, 22,51°±0,2°, 23,06°±0,2°, 24,78°±0,2°.

3. Kristalline Form I nach Anspruch 1 oder 2, **dadurch gekennzeichnet, dass** das Röntgenpulverbeugungsmuster der kristallinen Form I auch Beugungspeaks bei wenigstens einem der folgenden 2θ-Winkel aufweist: 12,25°±0,2°, 13,70°±0,2°, 13,99°±0,2°, 20,65°+0,2°, 21,83°±0,2°, 21,95°±0,2°, 24,59°±0,2°, 24,93°±0,2°, 25,77°±0,2°, 26,98°±0,2°.

4. Kristalline Form I nach irgendeinem der Ansprüche 1-3, **dadurch gekennzeichnet, dass** das Röntgenpulverbeugungsmuster der kristallinen Form I auch Beugungspeaks bei wenigstens einem der folgenden 2θ-Winkel aufweist: 13,58°±0,2°, 15,19°±0,2°, 18,00°±0,2°, 19,97°±0,2°, 23,16°±0,2°, 29,11°±0,2°, 29,33°±0,2°, 31,96°±0,2°, 32,42°±0,2°, 33,74°±0,2°.

5. Kristalline Form I nach irgendeinem der Ansprüche 1-4, **dadurch gekennzeichnet, dass** das Röntgenpulverbeugungsmuster der kristallinen Form I auch Beugungspeaks bei wenigstens einem der folgenden 2θ-Winkel aufweist: 10,71°±0,2°, 11,31°±0,2°, 20,32°±0,2°, 21,48°±0,2°, 23,31°±0,2°, 27,46°±0,2°, 27,75°±0,2°, 28,15°±0,2°, 29,82°±0,2°, 30,51°±0,2°, 30,83°±0,2°, 32,87°±0,2°, 34,78°±0,2°, 35,87°±0,2°, 36,25°±0,2°, 36,52°±0,2°, 37,33°±0,2°, 37,98°±0,2°, 38,43°±0,2°, 39,08°±0,2°, 39,34°±0,2°, 40,16°±0,2°, 40,78°±0,2°, 41,94°±0,2°, 42,18°±0,2°, 43,61°±0,2°, 44,35°±0,2°, 44,66°±0,2°, 45,39°±0,2°, 47,66°±0,2°.

6. Kristalline Form I nach irgendeinem der Ansprüche 1-4, **dadurch gekennzeichnet, dass** das Röntgenpulverbeugungsmuster der kristallinen Form I Beugungspeaks bei den folgenden 2θ-Winkeln aufweist: 6,89°±0,2°, 10,71°±0,2°, 12,25°±0,2°, 13,58°±0,2°, 13,70°±0,2°, 13,99°±0,2°, 14,45°±0,2°, 15,19°±0,2°, 16,42°±0,2°, 17,04°+0,2°, 18,00°±0,2°, 18,31°±0,2°, 19,35°±0,2°, 19,97°±0,2°, 20,32°±0,2°, 20,65°±0,2°, 21,83°±0,2°, 21,95°±0,2°, 22,25°±0,2°, 22,51°±0,2°, 23,06°±0,2°, 23,16°±0,2°, 23,31°±0,2°, 24,59°±0,2°, 24,78°±0,2°, 24,93°±0,2°, 25,77°±0,2°, 26,98°±0,2°, 27,46°±0,2°, 27,75°±0,2°, 29,11°±0,2°, 29,33°±0,2°, 30,51°±0,2°, 30,83°±0,2°, 31,96°±0,2°, 32,42°±0,2°, 33,74°±0,2°, 35,87°±0,2°, 36,25°±0,2°, 38,43°±0,2°, 40,16°±0,2°, 41,94°±0,2°, 42,18°±0,2°, 43,61°±0,2°.

7. Kristalline Form I nach irgendeinem der Ansprüche 1-4, **dadurch gekennzeichnet, dass** das Röntgenpulverbeugungsmuster der kristallinen Form I Beugungspeaks bei den folgenden 2θ-Winkeln aufweist: 6,89°±0,2°, 10,71°±0,2°, 11,31°±0,2°, 12,25°±0,2°, 13,58°±0,2°, 13,70°±0,2°, 13,99°±0,2°, 14,45°±0,2°, 15,19°±0,2°, 16,42°±0,2°, 17,04°+0,2°, 18,00°±0,2°, 18,31°±0,2°, 19,35°±0,2°, 19,97°±0,2°, 20,32°±0,2°, 20,65°±0,2°, 21,48°±0,2°, 21,83°±0,2°, 21,95°±0,2°, 22,25°±0,2°, 22,51°±0,2°, 23,06°±0,2°, 23,16°±0,2°, 23,31°±0,2°, 24,59°±0,2°, 24,78°±0,2°, 24,93°±0,2°, 25,77°±0,2°, 26,98°±0,2°, 27,46°±0,2°, 27,75°±0,2°, 28,15°±0,2°, 29,11°±0,2°, 29,33°±0,2°, 29,82°±0,2°, 30,51°±0,2°, 30,83°±0,2°, 31,96°±0,2°, 32,42°±0,2°, 32,87°±0,2°, 33,74°±0,2°, 34,77°±0,2°, 35,87°±0,2°, 36,25°±0,2°, 36,52°±0,2°, 37,33°±0,2°, 37,98°±0,2°, 38,43°±0,2°, 39,08°±0,2°, 39,34°±0,2°, 40,16°±0,2°, 40,78°±0,2°, 41,94°±0,2°, 42,18°±0,2°, 43,61°±0,2°, 44,35°±0,2°, 44,66°±0,2°, 45,39°±0,2°, 47,66°±0,2°.

8. Kristalline Form I nach irgendeinem der Ansprüche 1-4, **dadurch gekennzeichnet, dass** das Röntgenpulverbeugungsmuster der kristallinen Form I Beugungspeaks bei den folgenden 2θ-Winkeln aufweist: 6,89°±0,2°, 10,71°±0,2°, 11,31°±0,2°, 12,25°±0,2°, 13,58°±0,2°, 13,70°±0,2°, 13,99°±0,2°, 14,45°±0,2°, 15,19°±0,2°, 16,42°±0,2°, 17,04°+0,2°, 18,00°±0,2°, 18,31°±0,2°, 19,35°±0,2°, 19,97°±0,2°, 20,32°±0,2°, 20,65°±0,2°, 21,48°±0,2°, 21,83°±0,2°, 21,95°±0,2°, 22,25°±0,2°, 22,51°±0,2°, 23,06°±0,2°, 23,16°±0,2°, 23,31°±0,2°, 24,59°±0,2°, 24,78°±0,2°, 24,93°±0,2°, 25,77°±0,2°, 26,22°±0,2°, 26,98°±0,2°, 27,46°±0,2°, 27,75°±0,2°, 28,15°±0,2°, 29,11°±0,2°, 29,33°±0,2°, 29,82°±0,2°, 30,51°±0,2°, 30,83°±0,2°, 31,44°±0,2°, 31,96°±0,2°, 32,42°±0,2°, 32,87°±0,2°, 33,74°±0,2°, 34,77°±0,2°, 35,87°±0,2°, 36,25°±0,2°, 36,52°±0,2°, 37,33°±0,2°, 37,98°±0,2°, 38,43°±0,2°, 39,08°±0,2°, 39,34°±0,2°, 40,16°±0,2°, 40,78°±0,2°, 41,47°±0,2°, 41,94°±0,2°, 42,18°±0,2°, 42,71°±0,2°, 43,61°+%0,2°, 44,35°±0,2°, 44,66°±0,2°, 45,05°±0,2°, 45,39°±0,2°, 45,88°±0,2°, 46,69°±0,2°, 47,21°±0,2°, 47,66°±0,2°, 48,32°±0,2°, 48,97°±0,2°, 49,38°±0,2°, 50,35°±0,2°, 50,65°±0,2°, 51,97°±0,2°, 53,24°±0,2°, 55,00°±0,2°, 56,23°±0,2°, 58,12°±0,2°.

9. Kristalline Form I nach irgendeinem der Ansprüche 1-7, **dadurch gekennzeichnet, dass** das das Differentialscanningkalorimetriediagramm der kristallinen Form I einen endothermen Peak bei 158,49°C ± 3°C umfasst, wobei das Differentialscanningkalorimetriediagramm von Raumtemperatur bis etwa 300°C unter Verwendung einer linearen Heizvorrichtung bei 10°C/min erhalten wurde.

10. Verfahren zum Herstellen der kristallinen Form I der Verbindung mit Formel (I) nach irgendeinem der Ansprüche 1-9, umfassend:
Schritt 1): die Verbindung mit Formula (I) wird zu Lösungsmittel 1 zugegeben, und die resultierende Suspension wird gerührt, bis sie sich aufgelöst hat;
Schritt 2): Lösungsmittel 2 wird tropfenweise zu der klaren Lösung in Schritt 1) zugegeben; und
Schritt 3): das resultierende Gemisch wird gerührt und kristallisiert.

11. Verfahren nach Anspruch 10, **dadurch gekennzeichnet, dass** das Lösungsmittel 1 Ethylacetat, n-Propylacetat, Isopropylacetat, tert-Butylacetat, Methylacetat, Methanol, Ethanol oder Isopropanol ist.

12. Verfahren nach Anspruch 10 oder 11, **dadurch gekennzeichnet, dass** das Lösungsmittel 2 n-Hexan, Cyclohexan, n-Heptan, Xylol, Toluol oder Wasser ist.

13. Verfahren nach irgendeinem der Ansprüche 10-12, **dadurch gekennzeichnet, dass** das Lösungsmittel 1 Isopropylacetat ist und das Lösungsmittel 2 n-Heptan oder Toluol ist; oder das Lösungsmittel 1 Ethanol ist und das Lösungsmittel 2 Wasser ist.

14. Verfahren nach irgendeinem der Ansprüche 10-13, **dadurch gekennzeichnet, dass** in Schritt 1) die Suspension bei Raumtemperatur gerührt oder auf eine bestimmte Temperatur erwärmt wird, bis sie klar ist, wobei die bestimmte Temperatur 40°C-80°C beträgt.

15. Verfahren nach irgendeinem der Ansprüche 10-14, **dadurch gekennzeichnet, dass** in Schritt 3) das Rühren und die Kristallisation bei Raumtemperatur oder bei einer bestimmten Temperatur durchgeführt werden, wobei die bestimmte Temperatur 40°C-80°C beträgt.

16. Pharmazeutische Zusammensetzung, umfassend die kristalline Form I nach irgendeinem der Ansprüche 1-9 und ein(en) pharmazeutisch akzeptable(n/s) Träger, Hilfsstoff, Verdünnungsmittel, Adjuvans oder eine Kombination davon.

17. Pharmazeutische Zusammensetzung nach Anspruch 16, ferner umfassend einen oder mehrere andere aktive Inhaltsstoffe, ausgewählt aus SGLT-2-Inhibitoren, sGC-Aktivatoren, sGC-Stimulatoren, ACE-Inhibitoren, Renin-Inhibitoren, Angiotensin II-Rezeptor-Antagonisten, β-Blockern, Acetylsalicylsäure, Diuretika, Calcium-Antagonisten-Mitteln, Statinen, Digitalis-Derivaten, Calcium-Sensibilisatoren, Nitraten und Antithrombotika.

18. Kristalline Form I nach irgendeinem der Ansprüche 1-9 oder pharmazeutische Zusammensetzung nach irgendeinem der Ansprüche 16-17 zur Verwendung bei der Behandlung, Prävention oder Linderung der folgenden Erkrankung bei Patienten: diabetische Nephropathie, Hyperaldosteronismus, Hypertonie, Herzinsuffizienz, Folgeerscheinungen von Myokardinfarkt, Leberzirrhose, nichtalkoholische Steatohepatitis, chronische Nierenerkrankung, Fibrose, Niereninsuffizienz oder Schlaganfall.

## Revendications

1. Forme cristalline I du composé présentant la Formule (I) : 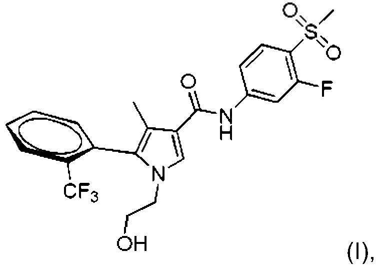 **caractérisée en ce que** le diagramme de diffraction des rayons X sur poudre de la forme cristalline I présente des pics de diffraction aux angles 2θ suivants : 14,45°± 0,2°, 17,04°± 0,2°, 19,35°± 0,2°, 22,51°± 0,2°, 24,78°± 0,2° dans lequel le diagramme de diffraction des rayons X sur poudre a été obtenu en utilisant un rayonnement Cu-Kα.

2. Forme cristalline I selon la revendication 1, **caractérisée en ce que** le diagramme de diffraction des rayons X sur poudre de la forme cristalline I présente des pics de diffraction aux angles 2θ suivants : 6,89°±0,2°, 14,45°±0,2°, 16,42°± 0,2°, 17,04°± 0,2°, 18,31°± 0,2°, 19,35°± 0,2°, 22,25°± 0,2°, 22,51°± 0,2°, 23,06°± 0,2°, 24,78°± 0,2°.

3. Forme cristalline I selon la revendication 1 ou 2, **caractérisée en ce que** le diagramme de diffraction des rayons X sur poudre de la forme cristalline I présente également des pics de diffraction à au moins un des angles 2θ suivants : 12,25°±0,2°, 13,70°±0,2°, 13,99°± 0,2°, 20,65°± 0,2°, 21,83°± 0,2°, 21,95°± 0,2°, 24,59°± 0,2°, 24,93°± 0,2°, 25,77°± 0,2°, 26,98°±0,2°.

4. Forme cristalline I selon l'une quelconque des revendications 1 à 3, **caractérisée en ce que** le diagramme de diffraction des rayons X sur poudre de la forme cristalline I présente également des pics de diffraction à au moins un des angles 2θ suivants : 13,58°±0,2°, 15,19°±0,2°, 18,00°± 0,2°, 19,97°± 0,2°, 23,16°± 0,2°, 29,11°± 0,2°, 29,33°± 0,2°, 31,96°± 0,2°, 32,42°±0,2°, 33,74°±0,2°.

5. Forme cristalline I selon l'une quelconque des revendications 1 à 4, **caractérisée en ce que** le diagramme de diffraction des rayons X sur poudre de la forme cristalline I présente également des pics de diffraction à au moins un des angles 2θ suivants : 10,71°± 0,2°, 11,31°± 0,2°, 20,32°± 0,2°, 21,48°± 0,2°, 23,31 °± 0,2°, 27,46°± 0,2°, 27,75°± 0,2°, 28,15°± 0,2°, 29,82°± 0,2°, 30,51°± 0,2°, 30,83°± 0,2°, 32,87°± 0,2°, 34,78°± 0,2°, 35,87°± 0,2°, 36,25°± 0,2°, 36,52°± 0,2°, 37,33°± 0,2°, 37,98°± 0,2°, 38,43°± 0,2°, 39,08°± 0,2°, 39,34°± 0,2°, 40,16°± 0,2°, 40,78°± 0,2°, 41,94°± 0,2°, 42,18°± 0,2°, 43,61 °± 0,2°, 44,35°± 0,2°, 44,66°± 0,2°, 45,39°± 0,2°, 47,66°± 0,2°.

6. Forme cristalline I selon l'une quelconque des revendications 1 à 4, **caractérisée en ce que** le diagramme de diffraction des rayons X sur poudre de la forme cristalline I présente des pics de diffraction aux angles 2θ suivants : 6,89°± 0,2°, 10,71°± 0,2°, 12,25°±0,2°, 13,58°±0,2°, 13,70°±0,2°, 13,99°±0,2°, 14,45°±0,2°, 15,19°±0,2°, 16,42°±0,2°, 17,04°±0,2°, 18,00°± 0,2°, 18,31°± 0,2°, 19,35°± 0,2°, 19,97°± 0,2°, 20,32°± 0,2°, 20,65°± 0,2°, 21,83°± 0,2°, 21,95°± 0,2°, 22,25°± 0,2°, 22,51°± 0,2°, 23,06°± 0,2°, 23,16°± 0,2°, 23,31°± 0,2°, 24,59°± 0,2°, 24,78°± 0,2°, 24,93°± 0,2°, 25,77°± 0,2°, 26,98°± 0,2°, 27,46°± 0,2°, 27,75°± 0,2°, 29,11 °± 0,2°, 29,33°± 0,2°, 30,51°± 0,2°, 30,83°± 0,2°, 31,96°± 0,2°, 32,42°± 0,2°, 33,74°± 0,2°, 35,87°± 0,2°, 36,25°± 0,2°, 38,43°± 0,2°, 40,16°± 0,2°, 41,94°± 0,2°, 42,18°±0,2°, 43,61°±0,2°.

7. Forme cristalline I selon l'une quelconque des revendications 1 à 4, **caractérisée en ce que** le diagramme de diffraction des rayons X sur poudre de la forme cristalline I présente des pics de diffraction aux angles 2θ suivants : 6,89°± 0,2°, 10,71°± 0,2°, 11,31°±0,2°, 12,25°±0,2°, 13,58°±0,2°, 13,70°±0,2°, 13,99°±0,2°, 14,45°±0,2°, 15,19°±0,2°, 16,42°±0,2°, 17,04°± 0,2°, 18,00°± 0,2°, 18,31°± 0,2°, 19,35°± 0,2°, 19,97°± 0,2°, 20,32°± 0,2°, 20,65°± 0,2°, 21,48°± 0,2°, 21,83°± 0,2°, 21,95°± 0,2°, 22,25°± 0,2°, 22,51°± 0,2°, 23,06°± 0,2°, 23,16°± 0,2°, 23,31°± 0,2°, 24,59°± 0,2°, 24,78°± 0,2°, 24,93°± 0,2°, 25,77°± 0,2°, 26,98°± 0,2°, 27,46°± 0,2°, 27,75°± 0,2°, 28,15°± 0,2°, 29,11°± 0,2°, 29,33°± 0,2°, 29,82°± 0,2°, 30,51°± 0,2°, 30,83°± 0,2°, 31,96°± 0,2°, 32,42°± 0,2°, 32,87°± 0,2°, 33,74°± 0,2°, 34,77°± 0,2°, 35,87°± 0,2°, 36,25°± 0,2°, 36,52°± 0,2°, 37,33°± 0,2°, 37,98°± 0,2°, 38,43°± 0,2°, 39,08°± 0,2°, 39,34°± 0,2°, 40,16°± 0,2°, 40,78°± 0,2°, 41,94°± 0,2°, 42,18°± 0,2°, 43,61 °± 0,2°, 44,35°± 0,2°, 44,66°± 0,2°, 45,39°±0,2°, 47,66°±0,2°.

8. Forme cristalline I selon l'une quelconque des revendications 1 à 4, **caractérisée en ce que** le diagramme de diffraction des rayons X sur poudre de la forme cristalline I présente des pics de diffraction aux angles 2θ suivants : 6,89°± 0,2°, 10,71°± 0,2°, 11,31°±0,2°, 12,25°±0,2°, 13,58°±0,2°, 13,70°±0,2°, 13,99°±0,2°, 14,45°±0,2°, 15,19°±0,2°, 16,42°±0,2°, 17,04°± 0,2°, 18,00°± 0,2°, 18,31°± 0,2°, 19,35°± 0,2°, 19,97°± 0,2°, 20,32°± 0,2°, 20,65°± 0,2°, 21,48°± 0,2°, 21,83°± 0,2°, 21,95°± 0,2°, 22,25°± 0,2°, 22,51°± 0,2°, 23,06°± 0,2°, 23,16°± 0,2°, 23,31°± 0,2°, 24,59°± 0,2°, 24,78°± 0,2°, 24,93°± 0,2°, 25,77°± 0,2°, 26,22°± 0,2°, 26,98°± 0,2°, 27,46°± 0,2°, 27,75°± 0,2°, 28,15°±0,2°, 29,11°± 0,2°, 29,33°± 0,2°, 29,82°± 0,2°, 30,51°± 0,2°, 30,83°± 0,2°, 31,44°± 0,2°, 31,96°± 0,2°, 32,42°± 0,2°, 32,87°± 0,2°, 33,74°± 0,2°, 34,77°± 0,2°, 35,87°± 0,2°, 36,25°± 0,2°, 36,52°± 0,2°, 37,33°± 0,2°, 37,98°± 0,2°, 38,43°± 0,2°, 39,08°± 0,2°, 39,34°± 0,2°, 40,16°± 0,2°, 40,78°± 0,2°, 41,47°± 0,2°, 41,94°± 0,2°, 42,18°±0,2°, 42,71°± 0,2°, 43,61 °± 0,2°, 44,35°± 0,2°, 44,66°± 0,2°, 45,05°± 0,2°, 45,39°± 0,2°, 45,88°± 0,2°, 46,69°±0,2°, 47,21°± 0,2°, 47,66°± 0,2°, 48,32°± 0,2°, 48,97°± 0,2°, 49,38°± 0,2°, 50,35°± 0,2°, 50,65°± 0,2°, 51,97°±0,2°, 53,24°± 0,2°, 55,00°± 0,2°, 56,23°± 0,2°, 58,12°± 0,2°.

9. Forme cristalline I selon l'une quelconque des revendications 1 à 7, **caractérisée en ce que** le diagramme de calorimétrie différentielle à balayage de la forme cristalline I comprend un pic endothermique à 158,49 °C ± 3 °C, dans lequel le diagramme de calorimétrie différentielle à balayage a été obtenu à partir de la température ambiante jusqu'à environ 300 °C en utilisant un dispositif de chauffage linéaire à 10 °C/min.

10. Procédé de préparation de la forme cristalline I du composé présentant la Formule (I) selon l'une quelconque des revendications 1 à 9, comprenant
étape 1) : le composé présentant la Formule (I) est ajouté dans le solvant 1, et la suspension résultante est agitée jusqu'à dissolution ;
étape 2) : le solvant 2 est ajouté goutte à goutte à la solution claire à l'étape 1) ; et,
étape 3) : le mélange résultant est agité et cristallisé.

11. Procédé selon la revendication 10, **caractérisé en ce que** le solvant 1 est l'acétate d'éthyle, l'acétate de n-propyle, l'acétate d'isopropyle, l'acétate de tert-butyle, l'acétate de méthyle, le méthanol, l'éthanol ou l'isopropanol.

12. Procédé selon la revendication 10 ou 11, **caractérisé en ce que** le solvant 2 est le n-hexane, le cyclohexane, le n-heptane, le xylène, le toluène ou l'eau.

13. Procédé selon l'une quelconque des revendications 10 à 12, **caractérisé en ce que** le solvant 1 est l'acétate d'isopropyle, et le solvant 2 est le n-heptane ou le toluène ; ou, le solvant 1 est l'éthanol, et le solvant 2 est l'eau.

14. Procédé selon l'une quelconque des revendications 10 à 13, **caractérisé en ce que**, dans l'étape 1), la suspension est agitée à température ambiante ou chauffée à une certaine température jusqu'à ce qu'elle soit claire, dans lequel la certaine température est comprise de 40°C à 80°C.

15. Procédé selon l'une quelconque des revendications 10 à 14, **caractérisé en ce que**, dans l'étape 3), l'agitation et la cristallisation sont mises en œuvre à température ambiante ou à une certaine température, dans lequel la certaine température est comprise de 40°C à 80°C.

16. Composition pharmaceutique, comprenant la forme cristalline I selon l'une quelconque des revendications 1 à 9, et un support, excipient, diluant, adjuvant, tous pharmaceutiquement acceptables, ou une combinaison de ceux-ci.

17. Composition pharmaceutique selon la revendication 16, comprenant en outre un ou plusieurs autres principes actifs choisis parmi les inhibiteurs de SGLT-2, les activateurs de sGC, les stimulateurs de sGC, les inhibiteurs de l'ACE, les inhibiteurs de la rénine, les antagonistes des récepteurs de l'angiotensine II, les β-bloquants, l'acide acétylsalicylique, les diurétiques, les agents antagonistes du calcium, les statines, les dérivés de digitaliques, les sensibilisateurs du calcium, les nitrates et les antithrombotiques.

18. Forme cristalline I selon l'une quelconque des revendications 1 à 9 ou composition pharmaceutique selon l'une quelconque des revendications 16 à 17 pour une utilisation dans le traitement, la prévention ou le soulagement de la maladie suivante chez des patients : néphropathie diabétique, hyperaldostéronisme, hypertension, insuffisance cardiaque, séquelles d'infarctus du myocarde, cirrhose du foie, stéatohépatite non alcoolique, maladie rénale chronique, fibrose, insuffisance rénale, ou accident vasculaire cérébral.
